# EUROPEAN PATENT SPECIFICATION

(11) **EP 1 117 357 B1**
(45) Date of publication and mention of the grant of the patent: **26.06.2013**
(21) Application number: 99951669.3
(22) Date of filing: 29.09.1999
(51) Int. Cl.: A61F 7/03, A61K 9/00, A61M 31/00, A61K 9/70, A61N 1/30

(54) **APPARATUS FOR IMPROVED ADMINISTRATION OF PHARMACEUTICALLY ACTIVE COMPOUNDS**
VORRICHTUNG FÜR VERBESSERTE VERABREICHUNG VON PHARMAZEUTISCH AKTIVEN STOFFEN
DISPOSITIFS SERVANT À AMELIORER L'ADMINISTRATION DE COMPOSES ACTIFS SUR LE PLAN PHARMACEUTIQUE

(30) Priority: 29.09.1998 US 162890; 24.05.1999 US 317313; 24.05.1999 US 317372
(43) Date of publication of application: 25.07.2001
(73) Proprietor: Nuvo Research Inc., Mississauga, Ontario L4T 4H4 (US)
(72) Inventor: ZHANG, Jie, Salt Lake City, UT 84121 (US); ZHANG, Hao, Midvale, UT 84047 (US)
(74) Representative: Gemmell, Peter Alan
(86) International application number: PCT/US1999/022698
(87) International publication number: WO 2000/018339

(56) References cited:
- EP-A- 0 304 227
- WO-A-85/02124
- WO-A-93/07842
- WO-A-93/07870
- WO-A-98/00118
- US-A- 4 230 105
- US-A- 4 286 592
- US-A- 4 685 911
- US-A- 4 898 592
- US-A- 5 298 257
- US-A- 5 658 583
- US-A- 5 679 373
- US-A- 5 730 721
- US-A- 5 919 479

## Description

### BACKGROUND OF THE INVENTION

Field of the Invention: The present invention relates to apparatus for administration of drugs. More particularly, the present invention relates to using controlled heat and other physical means to improve dermal, mucosal, and injection administration of drugs. The current invention is also related to novel designs and methods for manufacturing the heating devices used to generate heat by oxidation reaction for controlled heating.

State of the Art: The dermal administration of pharmaceutically active compounds involves the direct application of a pharmaceutically active formulation(s) to the skin, wherein the skin absorbs a portion of the pharmaceutically active compound which is then taken up by the blood stream. Such administration has long been known in the practice of medicine and continues to be an important technique in the delivery of pharmaceutically active compounds. For example, U.S. Patent 4,286,592 issued September 1,1981 to Chandrasekaran shows a bandage for administering drugs to a user's skin consisting of an impermeable backing layer, a drug reservoir layer composed of a drug and a carrier, and a contact adhesive layer by which the bandage is affixed to the skin.

Such dermal administration offers many important advantages over other delivery techniques, such as injection, oral tablets and capsules. These advantages include being noninvasive (thus, less risk of infection), avoiding first pass metabolism (metabolism of the drug in the liver when the drug is taken orally and absorbed through the gastrointestinal tract), and avoiding of high peaks and low valleys of concentration of pharmaceutically active compounds in a patient's bloodstream. In particular, unregulated high peaks and low valleys of concentration are typical in injection and oral administrations and are often associated with undesirable side effects and/or less than satisfactory intended effects.

The term "dermal drug delivery system" or "DDDS", as used herein, is defined as an article or apparatus containingpharmaceutically active compound(s) for delivery into the skin, the regional tissues under the skin, the systemic circulation, or other targeting site(s) in a human body via skin permeation. The term "DDDS" in this application, unless otherwise specified, only refer to those systems in which the main driving force for drug permeation is the drug concentration gradient.

The term "skin", as used herein, is defined to include stratum corneum covered skin and mucosal membranes.

The term "drug", as used herein, is defined to include any pharmaceutically active compound including but not limited to compounds that treat diseases, injuries, undesirable symptoms, and improve or maintain health.

The terms "targeted area" or "targeted areas", as used herein, are defined to include a systemic bloodstream of a human body, areas of a human body which can be reached by a systemic bloodstream including, but not limited to muscles, brain, liver, kidneys, etc., and body tissue regions proximate a location of an administered drug.

In DDDSs, a drug(s) is usually contained in a formulation, such as a hydro-alcohol gel, and may include a rate limiting membrane between the formulation and skin for minimizing the variation in the permeation of the drug. When a DDDS is applied to skin, the drug begins to transport out of the formulation, and transport across the rate limiting membrane (if present). The drug then enters the skin, enters blood vessels and tissues under the skin, and is taken into the systemic circulation of the body by the blood. At least some DDDSs have certain amount of pharmaceutically active compound in or on the skin side of the rate limiting membrane (if present) prior to use. In those DDDSs, that portion of the drug on the skin side of the rate limiting membrane will enter the skin without passing through the rate limiting membrane. For many drugs, a significant portion of the dermally absorbed drug is stored in the skin and/or tissues under the skin (hereinafter referred as "depot sites") before being gradually taken into the systemic circulation (hereinafter referred as "depot effect"). This depot effect is believed to be at least partially responsible for the delayed appearance of the drug in the systemic circulation after the application of some DDDSs and for continued delivery of the drug into the systemic circulation after the removal of some DDDSs from the skin.

In recent years there has been an increased interest in noninvasive androgen drug delivery systems. Dermal delivery systems are among those being developed, for such things as androgen replacement therapy. The major goals of testosterone replacement therapy are to restore serum testosterone concentrations to within the normal range for healthy men and, if possible, in a way that mimics the normal circadian pattern of endogenous secretion. More specifically it is desirable for the therapy to mimic the natural rise of testosterone level which peaks in the morning followed by gradual decrease, reaching a valley in the evening. Use of a androgen transdermal delivery system to deliver testosterone as disclosed in the present invention in hypogonadal men can achieve this goal. Other therapeutic uses of androgen(s) with the present invention include but are not limited to treatment of hypopituitarism, osteoporosis, menstrual disorders, refractory anemia, promotion of anabolism, and influencing conditions related to puberty.

Male hypogonadism is a disorder whereby testosterone production is reduced below the normal range of 3 to 10 mg/day. Symptoms of this disorder include impairment in:libido, sexual function, energy, mood, as well as regression of secondary sex characteristics and decreases in lean body mass and bone density. Available androgen replacement modalities include intramuscular injection of long-acting testosterone esters and oral administration of alkylated and esterified testosterone. However, neither of these treatments delivers testosterone in a manner which produces plasma levels mimicking normal circadian profiles of the endogenous hormone. Recently several transdermal testosterone systems have been developed. These systems have normalized serum testosterone concentrations over a period of 24 hours and allowed some approximation of the circadian pattern seen in healthy young men. Although these systems have proven useful, they are not without side effects. For example approximately 53 percent of men experience local skin reactions (contact dermatitis) at the application sites after using Androderm®, a testosterone patch, which in some instances necessitates discontinuing use of the patch.

The term "androgen transdermal therapeutic system" or "ATTS," as used herein, is defined as an article, apparatus or method for delivery of androgen into the human body via skin permeation. An ATTS is designed for therapeutic and other uses of androgens. The term "ATTS" in this application, unless otherwise specified, only refers to those systems in which the main driving force for drug permeation is the drug concentration gradient.

The term "androgen," as used herein, is broadly defined to include any pharmaceutically active compound which is capable of regulating masculine secondary sexual characteristics, including but not limited to esters of testosterone such as propionate, phenylacetate, enanthate, cypionate, methyl testosterone, fluoxymesterone, methandrostenolone, 17 alpha-methylnortestosterone, norethandrolone, stanolone, oxymetholone, stanozolol, ethylestrenol.

Additionally, androgens include pharmaceutically active agents which promote growth, such as an increase in height and development of skeletal musculature, thickening of the skin, proliferation of sebaceous glands, as well as loss of subcutaneous fat, growth of axillary and body hair, growth of the larynx, growth of beard and initiating the onset of male pattern baldness. Androgens may also be generally described as pharmaceutical agents acting on the pituitary, testes and sebaceous glands or an agent which has nitrogen retaining anabolic effects.

After placing a DDDS on the skin, the drug concentration in the targeted tissue or blood typically remains at or near zero for a period of time, before starting to gradually increase and reach a concentration deemed to be medicinally beneficial, called the "therapeutic level" (the time it takes to reach the therapeutic level is referred to hereinafter as the "onset time"). Ideally, the concentration of the drug in the targeted tissue or blood should plateau (i.e., reach a substantially steady state) at a level slightly higher than the therapeutic level and should remain there for extended period of time. For a given person and a given DDDS, the "concentration of the drug in the targeted tissue or bloodstream vs. time" relationship usually cannot be altered under normal application conditions.

The onset time and the delivery rate of the drug into the targeted area(s) of the body for a typical DDDS are usually determined by several factors, including: the rate of release of the drug from the formulation, the permeability of the drug across the rate limiting membrane (if a rate limiting membrane is utilized), the permeability of the drug across the skin (especially the stratum corneum layer), drug storage in and release from the depot sites, the permeability of the walls of the blood vessels, and the circulation of blood and other body fluid in the tissues (including the skin) under and around the DDDS. Although these primary factors affecting onset time and delivery rate are known, no existing DDDS is designed to have an alterable delivery rate in the course of the application of the drug.

While a DDDS works well in many aspects, current dermal drug delivery technology has some serious limitations, including: 1) the onset time is undesirably long for many DDDSs; 2) the rate that the drug is taken into the systemic circulation or the targeted area(s) of the body cannot be easily varied once the DDDS is applied onto the skin and, when the steady state delivery rate is achieved, it cannot be easily changed; and 3) the skin permeability is so low that many drugs are excluded from dermal delivery because the amount of drug delivered is not high enough to reach a therapeutic level. In addition, temperature variations in the skin and the DDDS are believed contribute to the variation of dermal absorption of drugs.

It is known that elevated temperature can increase the absorption of drugs through the skin. U.S. Patent 4,898,592, issued February 6, 1990 to Latzke et al., relates to a device for the application of heated transdermally absorbable active substances which includes a carrier impregnated with a transdermally absorbable active substance and a support. The support is a laminate made up of one or more polymeric layers and optionally includes a heat conductive element. This heat conductive element is used for distribution of the patient's body heat such that absorption of the active substance is enhanced. U.S. Patent 4,230,105, issued October 28,1980 to Harwood, discloses a bandage with a drug and a heat-generating substance, preferably intermixed, to enhance the rate of absorption of the drug by a user's skin. Separate drug and heat-generating substance layers are also disclosed. U.S. Patent 4,685,911, issued August 11,1987 to Konno et al., discloses a skin patch including a drug component, and an optional heating element for melting the drug-containing formulation if body temperature is inadequate to do so.

Another area of administration involves delivering drugs in controlled/extended release form/formulations ("form/formulation") into the skin or tissues under the skin (the residing place for these form/formulations are hereinafter referred as "storage sites") which results in the drugs being released from the storage sites in a controlled/extended fashion. The most common technique to deliver the form/formulations into the storage sites is by injection. Other techniques may also be used, such as implantation and forcing the form/formulation into the skin with high-speed hitting. However, once the form/formulation is delivered into the storage sites, it is usually difficult to alter the rate, known as the "release rate", that the drug is released from the form/formulation at the storage sites, and taken into the systemic circulation or the targeted area(s) of the body.

Yet another area of administration involves injecting drugs subcutaneously or intramuscularly. In some clinical situations, it is beneficial to accelerate the speed of drug absorption into the systemic circulation or other targeted areas(s) in the body after such injection.

While it is known that elevated temperatures can increase the absorption of a drug through the skin, providing efficient, convenient, and controlled heat to improve dermal delivery is difficult. Moreover, in some applications or medical treatments, the use of a separate heating element in the administration of dermal drug delivery systems to increase the absorption of drugs through the skin may present a number of complications in dermal drug administration. For example, the use of a temperature control element can complicate the administration of the therapeutic agent by requiring the patient or care giver to take additional steps to employ the temperature control element, such as acquiring, storing and preparing the separate temperature control element and the administrating and removing the separate temperature control element.

Also, as the complexity of administrating the therapeutic agent increases, the likelihood of compliance by the patient or caregiver with the prescribed use of the temperature control element tends to decrease, potentially reducing the effectiveness of the prescribed treatment. If the prescribed use requires a patient to purchase, store, prepare, administer and then remove a separate heating element in addition to administering a DDDS, the patient may feel inconvenienced by the additional time and choose to forego the prescribed use of the separate temperature control element. Furthermore, the use of a separate temperature control element is limited by the compatibility between a given temperature control element and the DDDS with which the temperature control element is to be used. The shape, formulation and configuration of the DDDS may prevent effective use of a separate heating element, where the separate heating element is not specifically designed for use with the DDDS.

While there are disadvantages of attempting to resolve the difficulties ofusing a separate temperature control element with a DDDS by simply combining the two (without careful design consideration) is often problematic and unsuccessful. For example, one could attempt to combine the temperature control element with a DDDS by making the drug formulation itself capable of generating heat when exposed to oxygen or by another mechanism. However, in order to do so it would be necessary for the heat generating medium and the drug formulation to be completely compatible with each other. When using an exothermic oxidation reaction to generate heat, if the heat generating medium comprising iron powder, activated carbon and water is mixed with an aqueous gel-based local anesthetic formulation, it cannot generate heat properly because, among other reasons, the gel in the local anesthetic formulation would prevent oxygen from entering the heat generating medium.

Another approach which initially appears straightforward would be simply affixing a heating patch onto a drug patch, and placing the integrated patch into an air-tight container. This approach was utilized by Albert Argaud in U.S. Patent No. 4,963,360. The Argaud patent teaches the use of a base sheet to which is applied on one side a gelatin layer holding the medication, and on the other side a composition designed to have a exothermic reaction when exposed to air. Because there is no heat regulating mechanism in the Argaud patent, the absorption into the skin of the medicinal component will not be controlled. Uncontrolled absorption can cause serious reactions in patients due to drug overdose and under dose. These attendant side affects out weigh the benefits provided by the exothermic reaction. In addition to the problems of regulating the heat, other problems, such as the lack of any insolation or any engineering to direct the heat into the body, also reduce the effectiveness and consistency of the exothermic reaction in a DDDS.

Since these early delivery devices did not provide for a mechanism for sealing the medicinal layer against the skin, rapid evaporation of the medicinal component can occur once the gel is exposed to air. Moreover, without a means to affix the patch securely to the skin, there is no assurance of proper absorption. As can be seen by looking at the example provided by the Argaud reference, there is a limited contact area between the medicinal layer and the contact area is likely to vary, affecting the amount of drug absorbed. Another problem with the Argaud patch is that because of the packaging of the device, the air within the package is allowed to communicate with both the drug formulation and heat generating medium. This approach allows the exchange or transfer of substance(s) between the heat generating medium and the drug formulation during storage, which may compromise either or both the drug formulation and the heat generating medium. For instance, if the heat generating medium has a proper ratio of iron powder, activated carbon, salt, wood powder and water and the drug formulation is in the form of a hydrogel, the heat generating medium may absorb water vapor from the drug formulation, and thus change the desired concentrations of water in both the heat generating medium and the drug formulation. This problem as it applies to the use of drugs such as fentanyl is explained in greater detail below.

The difficulty of combining a temperature control unit with a DDDS is illustrated by the following example of combining a heating oxidation patch with a fentanyl DDDS. By affixing a heating component having a heat generating medium as described in the paragraph above disposed to a drug patch having a formulation containing alcohol and water, one could attempt to form an integrated patch, and this integrated patch could be sealed in an air-tight container. Although the air-tight container would separate the integrated patch from the outside environment, and although a barrier film may be placed between the heating component and the drug formulation, alcohol and water in the drug formulation could still migrate into the space in the air-tight container in the form of vapors and be absorbed into the heat generating medium. The activated carbon in the heat generating medium has a strong tendency to absorb volatile substances. Therefore over time, the drug formulation would lose a significant amount of alcohol and water.

Both alcohol and water play very important roles in the transdermal delivery of some drugs. At least one function of alcohol in the formulation is to increase skin permeability, so that the desired amount of drug can be absorbed. Water and alcohol also serve as the solvent of the drug in the formulation. If a temperature control apparatus and a DDDS are combined as explained in the paragraph above, significant amounts of alcohol and water would be lost during storage and skin permeability would not be increased as designed, leading to lower dermal absorption of the drug. In addition, drug solubility and concentration in the formulation would be changed, which would change the driving force for transdermal drug permeation. As a result, drug absorption from the transdermal patch would likely be quite different from the designed rates and be quite unpredictable. This could cause serious drug under dose or overdose.

Furthermore, if enough alcohol and water are absorbed into the heat generating medium, the function of the heating component may also be compromised. In a heat generating medium using activated carbon, the activated carbon has a tendency to absorb moisture from the surrounding environment. If the water quantity in the heat generating medium is increased too much, the heat generating medium will not generate heat properly. Thus it is important to shield the heat generating medium from moisture. It is similarly important to protect the heat generating medium from exposure to oxygen to prevent the oxidation reaction from transpiring prematurely.

Thus, it is very important to have good separation between the drug formulation and the heating component in an integrated patch, even if the integrated patch is sealed in an air-tight container. This separation should not only prevent direct transfer of substance(s) between the drug formulation and the heating component (i.e., permeation) but also prevents the transfer or exchange through vapor via the space in the airtight container.

Heating devices for heating human skin are plentiful in the art. The heating element used in a heating device has a significant impact on the design and overall performance of the heating device. As a heat generating medium, the use of elements capable of undergoing an exothermic oxidation reaction has the advantage of being controlled by exposing the oxidation reaction elements to ambient oxygen. For example, an oxidation-based, heat- generating hand warmer may comprise an air-permeable bag containing a heat generating medium. The mixture may comprise loose granules of iron powder, activated carbon, water, salt, and optionally a material such as wood powder for making the medium more porous. The hand warmer is usually stored in an airtight container. After it is taken out of the container, oxygen in ambient air flows into the heat generating medium through the air-permeable bag, and the exothermic oxidation of iron powder in the heat generating medium starts to generate heat.

With apparatus designed for warming the hand or body, the heating devices are not usually manufactured to be compact, and the heating temperature and duration of heat generated are not designed to be precisely controlled. For example, the hand warmer distributed by GRABBER Warmers, Grand Rapids, MI 49512 has minimum and maximum temperatures of 40°C and 69 °C, respectively, and weighs about 20 grams. However, in some situations the size of the heating device and the ability to control temperature and duration of the heat may be important.

US Pat. No. 5,658,583 as closest prior art discloses oxidation-reaction based devices to generate heat for enhancing dermal drug delivery. A heat generating device as disclosed in the patent is a thin, flexible chamber defined by a bottom and surrounding walls made of materials non-permeable to air, and a cover with a structure which allows oxygen in ambient air to flow into the chamber at a proper rate. Inside the thin, flexible chamber is a heat generating medium capable of generating heat when exposed to oxygen. A typical composition of the heat generating medium include activated carbon, iron powder, sodium chloride, fine wood powder, and water in a proper ratio.

In many medical related applications, such as enhancing transdermal drug delivery and regulating injected or implanted controlled drug release systems, the heating device must meet certain criteria for the device to be functional and practical. For example, the device usually needs to be thin and compact. The duration and temperature of the heat generated need to be precisely controlled and reproducible, so that the risk of drug overdose or under dose can be minimized. Additionally it is often desirable to be able to place as much heat generating medium into the chamber as possible, so that the heating device, while compact, can generate heat for sufficient duration. Moreover, the device may need to be sterile and disposable. The design of the heat generating medium affects the potential applications of the heating device.

These limitations in design can pose serious problems for certain applications, such as in many medically related applications, and when the volume of the chamber is designed to be small.

Therefore, it would be advantageous to develop methods and apparatus to improve the drug administration of DDDSs, and, more specifically, to make the use of DDDSs more flexible, controllable, and titratable (varying the drug delivery rate, amount, or period according to the biological effect of the drug) to better accommodate various clinical needs. It would also be advantageous to develop methods and apparatus to make dermal delivery possible for drugs which are currently excluded because of low skin permeability. It would further be advantageous to develop means to alter mainly to increase the drug absorption rate from the storage sites or injection sites in such ways that can accommodate certain clinical needs.

Furthermore, it would be advantageous to develop methods and apparatus to improve the androgen administration of ATTSs, to better accommodate various clinical needs, and to minimize side effects. For example it would be advantageous to develop a drug delivery system that can elevate skin temperature to a desired temperature range. The desired temperature range should be a range which improves the administration of the androgen, but does not significantly increase the chances of trauma to the skin due to overheating. Similarly it would be advantageous to provide elevated temperatures within a prescribed range which can be altered or adjusted within the range as needed. Having an adjustable temperature would allow a patient or caregiver greater control over the absorption rate. Furthermore it would also be advantageous to provide an elevated temperature for a controlled period of time or a desired duration. It would also be advantageous to develop a method and apparatus to allow the patient or caregiver to freely select the site on the skin where temperature is to be elevated.

It would also be an advancement in the art to provide a configuration that combines the convenience and ease of use of an integrated temperature control component with a dermal drug delivery component that can simultaneously prevent undesired transfer of substance(s) between the temperature control component and dermal drug delivery system, shields them as necessary from ambient oxygen and undesired solvents, and prevents undesired gain or loss of the solvent to the environment.

### SUMMARY OF THE INVENTION

The invention is defined in appended independent claims 1 and 10. Preferred embodiments are described in the dependent claims.

In the application of a DDDS, the absorption of the drug is usually determined by a number of factors including: the diffusion coefficient of drug molecules in the drug formulation, the permeability coefficient of the drug across the rate limiting membrane (if one is used in the DDDS), the concentration of dissolved drug in the formulation, the skin permeability of the drug, drug storage in and release from the depot sites, the body fluid (including blood) circulation in the skin and/or other tissues under the skin, and permeability of the walls of capillary blood vessels in the sub-skin tissues. Thus, in order to address the limitations of the current dermal drug delivery technologies, it is desirable to have control over and have the capability to alter these drug absorption factors. It is believed that controlled heating/cooling can potentially affect each one of the above factors.

Specifically, increased temperature generally can increase diffusion coefficients of the drugs in the formulations and their permeability across the rate limiting membrane and skin. Increased heat also increases the blood and/or other body fluid flow in the tissues under the DDDS, which should carry the drug molecules into the systemic circulation at faster rates. Additionally, increased temperature also increases the permeability of the walls of the capillary blood vessels in the sub-skin tissues. Furthermore, increased temperature can increase the solubility of most, if not all, drugs in their formulations which, in formulations with undissolved drugs, should increase permeation driving force. Of course, cooling should have substantially the opposite effect. Thus, the present invention uses controlled heating/cooling to affect each of the above factors for obtaining controllable dermal absorption of drugs.

The present invention also uses controlled heating/cooling in several novel ways to make dermal drug delivery more flexible and more controllable in order to deal with various clinical conditions and to meet the needs of individual patients. More broadly, this invention provides novel methods and apparatus for controlled heating/cooling (hereinafter "temperature control apparatus") during the application of the DDDS, such that heating can be initiated, reduced, increased, and stopped to accommodate the needs.

Another embodiment of the present invention is to determine the duration of controlled heating on DDDS based on the effect of the drug for obtaining adequate amount of the extra drug and minimizing under-treatment and side effects associated with under and over dosing.

Through the proper selection, based on the specific application and/or the individual patient's need, of the moment(s) to initiate controlled heating, heating temperature, and moment(s) to stop the controlled heating, the following control/manipulation of the absorption rates should be achieved: 1) shorten the onset time of the drug in the DDDS without significantly changing its steady state delivery rates; 2) provide proper amount of extra drug during the application of a DDDS when needed; and 3) increase the drug absorption rate throughout a significant period of duration or throughout the entire duration of the DDDS application.

Shortening of onset time is important in situations where the DDDS provides adequate steady state deliver rates, but the onset is too slow. Providing the proper amount of extra drug is important where a DDDS delivers adequate "baseline" amount of the drug, but the patient needs extra drug at particular moment(s) for particular period(s) of time during the application of the DDDS. Increasing the drug absorption rate is used for the patients who need higher drug delivery rates from the DDDS.

The first of above approach can be achieved by applying controlled heating at the starting time of the DDDS application, and design the heating to last long enough to cause the concentration of the drug in the systemic circulation or other targeted area of the body to rise toward the therapeutic levels, and stops (may be gradually) shortly after that. The second approach may be achieved by applying controlled heat when a need to obtain extra drug are rises, and terminating the controlled heating either at a predetermined moment or when the desired effect of the extra drug is achieved. The third approach can be achieved by applying the controlled heat at the starting time of the DDDS application. In all those three approaches, temperature of the controlled heating needs to be designed to control the degree of increase in said that drug delivery rates.

Due to low skin permeability of the skin, onset times of conventional DDDSs are usually quite long, and often undesirably long. Thus, another aspect of the present invention is to provide methods and apparatus for using controlled heat to shorten the onset times of DDDSs, preferably without substantially changing the steady state drug delivery rates. A particularly useful application of this aspect of the present invention is to provide a controlled heating apparatus for use with conventional, commercially available DDDSs to shorten the onset times in clinical use, without having to re-design the DDDSs or adjust their steady state drug delivery rates.

It is believed that an important cause for variation in drug absorption in DDDSs is variation in temperature of the DDDSs and the adjacent skin caused by variations in ambient temperature and/or physical condition of the person. This temperature variation can, of course, potentially affect all of the factors that collectively determine the ultimate drug delivery rates of the DDDSs. Thus, the present invention of providing methods and apparatus to use controlled heating/cooling also minimizes the variation in temperature of the skin and the DDDSs applied on the skin. It is also contemplated that an insulating material can be incorporated with the controlled temperature apparatus to assist in not only minimizing the temperature variation, but also increasing the temperature of the DDDS and the skin under it (by decreasing heat loss), each of which tend to increase dermal drug absorption.

The present invention also relates to methods and apparatus for using an insulating device, such as a cover made of insulating material (such as closed-cell foam tape) with adhesive edges, and a size slightly larger than the DDDS or the area over an injected drug, to cover the DDDS/injected drug when the DDDS and/or the skin of the user is exposed to extreme temperature (such as a hot shower or bath, direct sunlight, etc.).

An important area in modem anesthesiology is patient controlled analgesia (hereinafter "PCA"), in which the patient gives himself a dose of analgesic when he feels the need. The ranges of the dose and dosing frequency are usually set by a care giver (*i.e.*, caring physician, nurse, etc.). In many PCA situations, the patient receives a baseline rate of analgesic, and gets extra bolus analgesic when he feels that it is needed. The technology in the present invention may be used for a PCA in which the patient gets the baseline dose by a regular dermal analgesic patch and the extra ("rescue") dose by heating the dermal analgesic patch. The heating temperature and duration needs to be designed to deliver a proper amount of extra dose.

Drugs in controlled or extended release forms or formulations may be delivered into depot/storage sites in the skin and/or the tissues under the skin with methods such as injection, implantation, hitting the drug/drug formulation on the skin with supersonic speed, and embedding the drug/drug formulation onto the skin. The controlled/extended form/formulation allows the drug to be released gradually into the surrounding tissues and/or systemic circulation over an extended period of time. For instance, extended release insulin (such as Ultralente^{®} zinc insulin - Eli Lilly and Co.) can be injected subcutaneously to deliver insulin into the patient's systemic circulation over an extended period of time. However, once the drug in the controlled/extended form/formulation is delivered to the storage sites, it is usually difficult to alter or control the course of drug release. The apparatus and methods of the present invention allow controlled heat to increase and controlled cooling to decrease, the drug release from the controlled/extended form/formulation after it is delivered into the depot/storage sites. For example, many diabetic patients need additional insulin shortly before meals to suppress the blood sugar increase resulting from the meals. However, the release rate of the subcutaneously injected extended release insulin is relatively constant.

With the methods and apparatus in the invention, a diabetic patient may inject a subcutaneous extended release insulin in the morning and apply controlled heat on the skin of the injection site for a duration of time shortly before ingestion of a meal to obtain additional insulin to suppress the sugar from the meal. The controlled heat increases the flow of blood and other body fluid surrounding the storage sites and is believed to increase the dissolution rate of insulin. It is, of course, understood that whether a given controlled/extended release formulation in the depot/storage sites can actually release extra drug with increased temperature depends on the nature of the drug form/formulation. However, since heat is known or expected to increase the diffusion speed of drugs in their formulations, increase the permeability of blood vessel walls, and increases the circulation of body fluid surrounding the depot sites, each of which tend to favor increased drug release, the heat-induced extra drug release is expected to take place for many, if not most, controlled/extended drug form/formulation delivered into sub-skin storage sites.

One important aspect of the present invention is to properly choose the temperature of the controlled heat and the moment(s) to initiate and stop the controlled heat in the applications with injected drug formulations, especially extended/controlled release formulations, to accommodate the needs of different therapies and individual patients, in ways similar to the applications with DDDSs discussed above.

Many biodegradable polymers may be used to make controlled/extended release formulations. Of particular note are the biogradable lactic/glycolic acid polymers described in Chapters 29 and 33 of Encyclopedic Handbook of Biomaterials and Bioengineering, edited by Donald L. Wise, et al., publ. Marcel Dekker, 1995. It is one important aspect of the present invention to use controlled heat, as discussed above, to control/regulate drug release rates from controlled/extended release formulations made with such polymers, and preferably, prepared using the methods described in the Encyclopedic Handbook of Biomaterials and Bioengineering.

For drugs where quick systemic absorption is important, the present invention may be beneficial. For example, it is generally agreed that to successfully treat a migraine headache, concentrations of an anti-migraine drug, such as dihydroergotamine, in the bloodstream must reach a therapeutic level within a certain time from the onset of migraine headache. In such situations, the heating devices, as discussed above, may be used with normal injection of drugs. Since heat can usually increase the diffusion speed of drugs in their formulations, increase the permeability of blood vessel walls, and increases the circulation of body fluid surrounding the injection site, the drug will enter the system circulation more quickly.

One of the more important aspects of the present invention is the apparatus for generating and providing controlled heating. These controlled heat generating apparatus generally comprise a heat generating portion and a means to pass the heat generated by the heat generating portion to the DDDSs, the skin, and/or the sub-skin depot and storage sites. These controlled heat generating apparatus generally further include a mechanism (such as tape, adhesive, and the like) for affixing apparatus onto the DDDSs and/or the skin. Preferably, the affixation mechanism securely holds the controlled heat generating apparatus in place while in use, but it also allows relatively easy removal after use. Additionally, these controlled heat generating apparatus may further include a mechanism for terminating the generation of heat. The shape and size of the bottom of the controlled heat generating apparatus are generally specially made to accommodate the DDDSs with which they are to be employed.

One embodiment of a controlled heat generating apparatus is a shallow chamber including non-air permeable side wall(s), a bottom wall, and a non-air permeable top wall which has area(s) with limited and desired air permeability (*e.g.*, holes covered with a microporous membrane). A heat generating medium is disposed within the shallow chamber. The heat generating medium preferably comprises a mixture of iron powder, activated carbon, salt, water, and, optionally, sawdust. The controlled heat generating apparatus is preferably stored in an air-tight container from which it is removed prior to use. After removal from the air-tight container, oxygen in the atmosphere ("ambient oxygen") flows into heat generating medium through the areas on the non-air permeable top with desired air-permeability to initiate a heat generating oxidation reaction (*i.e.*, an exothermic reaction). The desired heating temperature and duration can be obtained by selecting the air exposure of the top (*e.g.*, selecting the right size and number of holes on the cover and/or selecting the microporous membrane covering the holes for a specific air permeability), and/or by selecting the right quantities and/or ratios of components of the heat generating medium.

This embodiment of the controlled heat generating apparatus preferably includes a mechanism for affixing the controlled heat generating apparatus onto the skin or a DDDS that is applied to the skin. For applications where the removal or termination of the heating might be necessary, the heat generating apparatus may also have a mechanism for allowing easy removal from the DDDS and/or the skin or for termination of the heating. One mechanism for allowing easy removal of the shallow chamber from a DDDS without removing the latter from the skin comprises a layer of adhesive on the side walls of the heat generating apparatus with an non-adhesive area or less adhesive area (less adhesive than the adhesive affixing the DDDS to the skin) at the bottom of the shallow chamber, with the non- or less adhesive area having a shape similar to that of the DDDS. When such a heat generating apparatus is applied onto the DDDS which is on the skin, the adhesive at the bottom of the side walls of the heat generating apparatus adheres to the skin, and non- or less adhesive part is on top of, but not adhered or not strongly adhered to, the DDDS. This allows for removal of the heat generating apparatus without disturbing the DDDS.

Although one application of such a heat generating apparatus is to be used in conjunction with a DDDS, it is understood that the heat generating apparatus can also be applied directly to the skin to increase the release of drugs from depot sites or sites of injection or implantation of controlled released drugs (storage sites), or to accelerate the absorption of subcutaneously or intramuscularly injected drugs.

The heat generating mechanism of the present invention for the controlled heat generating apparatus is not limited to the preferred exothermic reaction mixture of iron powder, activated carbon, salt, water, and, optionally, sawdust, but may include a heating unit whose heat is generated by electricity. The electric heating unit, preferably, includes a two dimensional surface to pass the heat to the DDDS and/or the skin. The electric heating unit may also include a temperature feedback system and a temperature sensor that can be placed on the DDDS or the skin. The temperature sensor monitors the temperature at the DDDS or skin and transmits an electric signal based on the sensed temperature to a controller which regulates the electric current or voltage to the electric heating unit to keep the temperature at the DDDS or skin at desired levels. Preferably, a double sided adhesive tape can be used to affix the electric heating unit onto the skin.

The heat generating mechanism may also comprise an infrared generating unit and a mechanism to direct the infrared radiation onto the DDDS or the skin. It may also have a temperature feedback system and a temperature sensor that can be placed on the DDDS or the skin to control the intensity of the infrared emission to maintain the temperature at the DDDS or skin at desired levels.

The heat generating mechanism may further comprise a microwave generation unit and a mechanism to direct the microwave radiation onto the DDDS or the skin. Again, the heat generating mechanism may have a temperature feedback system and a temperature sensor to regulate the intensity of the microwave emission to maintain the temperature at the DDDS or skin at desired levels.

The heat generating mechanism may yet further comprise a container containing supercooled liquid which generates heat from crystallization ("exothermic"). The crystallization is initiated within the container, such as, by bending a metal piece in the supercooled liquid, and the container is placed on a DDDS or on the skin. The heat which is released from the crystallization process is passed to the DDDS and/or the skin. However, heat generated by crystallization usually does not maintain a constant level over extended time. Thus, such a heat generating mechanism is not ideal for applications where elevated temperature in a narrow range over an extended time is necessary, but is useful where only a short heating duration is needed, such as with a DDDS that would benefit from short heating duration to minimize the onset time.

Although, in general, most benefits for DDDSs are realized from increased drug absorption and release rates by heating, there are circumstances where it may be desirable to be able to both increase and decrease the drug absorption and release rates. It is understood that for a more complete control in dermal and controlled/extended release drug administration that a mechanism for providing both heating or cooling, depending on need, would be advantageous. Thus, a novel approach of this invention is to provide methods and apparatus for providing heating or cooling to the DDDSs, the skin and/or the tissues under it, or the controlled/extended release drug form/formulation in the skin or the tissues under the skin, such that the drug absorption and/or release can be controlled. The heating/cooling mechanism comprises a thermoelectric module which functions as a heat pump wherein the power supply may be reversed depending on whether heating or cooling is desired. A cooling mechanism can include an endothermic crystallization mechanism similar to the exothermic crystallization mechanism discussed above.

It is, of course, understood that the use of controlled heating and/or cooling to control drug absorption and/or release are equally applicable to controlled/extended form/formulations after they are delivered into the skin and/or tissues under the skin. However, physical mechanisms other than heating and/or cooling may also be used for the same purpose. Thus, it is novel approach of this invention to provide methods and apparatus to use ultrasound, electric current, and mechanical vibration to induce extra drug release from controlled/extended release form/formulations which are already delivered into the body and that are responsive to these physical induction means.

The present invention also provides for the integration of a drug delivery component, such as a transdermal drug delivery system, with a temperature control component, such as a heating patch to turn an "integrated DDDS patch or "integrated patch." The drug delivery component comprises a drug formulation applicator and a drug formulation secured to the drug formulation applicator. A barrier and/or compartment prevents undesired substance transfer between the temperature control component and the drug delivery component. A barrier and/or compartment also prevents exchange transfer or absorption of volatile substances between the drug delivery and temperature control components and the external environment. The temperature control component comprises a temperature modification element and a temperature control which can control or adjust the heat generated by the temperature modification element.

The drug delivery component may be similar to known dermal drug delivery systems having a drug disposed within a formulation, the formulation adhering to or contained within a drug applicator. A drug formulation applicator can be any structure or process in a dermal drug delivery system which facilitates or results in the delivery of the drug or drug formulation to the skin of a patient, for example, a gauze pad secured to adhesive tape. The drug applicator may include a rate limiting membrane between the drug formulation and the user's skin, or alternatively the formulation may be in direct contact with the skin. A physical barrier, such as an impermeable medical packaging film, provides means for preventing exchange or substance(s) between the drug delivery component and the temperature control component via direct permeation or vapor absorption. The drug applicator with the drug formulation are secured to the means for preventing exchange. This drug delivery component is integrated with the temperature control component to form the integrated patch. Additionally, a layer of medical adhesive tape may be secured to the barrier film or other part of the integrated patch, thereby providing means for attaching the integrated patch to the skin of the patient.

The absorption of the therapeutic drug for an integrated DDDS patch is usually determined by a number of factors including: the diffusion coefficient ofdrug molecules in the drug formulation, the permeability coefficient of the drug across a rate limiting membrane (if any), the concentration of dissolved drug in the formulation, the skin permeability to the drug, the body fluid (including blood) circulation in the skin and/or other tissue under the skin, permeability of the walls of capillary blood vessels in the sub-skin tissues and absorption into and release from depot sites in the sub-skin tissues. It is believed that controlled heating can potentially affect each one of the above factors, and thus, it is desirable and convenient to have a temperature control component integrated with a drug delivery component.

The integrated DDDS patch of the present invention provides for the use of a wide variety of drug formulations. The formulation itself may take various forms such as liquid, gel, cream, paste, or solid. Generally, a therapeutic agent is mixed or dissolved into the drug formulation. The drug delivery system of the present invention contemplates the use of a transdermally administered drug in a drug formulation including, but not limited to, drugs such as analgesics, androgens, anesthetics, and anesthetic agents. The drug formulation applicator is configured to hold the drug formulation such that the drug formulation on the applicator can be easily removed from its storage pocket and administered to a patient's skin.

The temperature control component has a temperature modification element which may be a heat generating element (for example, a heating patch) and a temperature control, to allow the user to adjust the temperature. Heating patches are specifically designed to improve the efficiency and therapeutic effectiveness of dermal drug delivery systems. An important feature of the heating patch is that it can quickly increase skin temperature to a temperature around 39°C - 43°C. The heating patch can maintain skin temperature in that range for an extended period of time. This not only provides consistent heating, but also prevents skin damage which could be caused by over heating when using other heating methods.

One embodiment of the heating patch is particularly useful for the integrated patch. The heating patch comprises a shallow chamber defined by a bottom, a frame wall, and a cover. Within the shallow chamber is a heat generating medium which, upon contact with ambient oxygen, can generate heat. The chamber has a cover which is made of a material impermeable to oxygen. The cover has areas which are open to allow oxygen into the chamber. The openings may be selectively covered, partially covered, or opened by the user to control air flow into the chamber and the heat generated therein.

Alternatively, certain or all areas of the cover may be covered by a membrane with certain permeability to air. Thus the cover can allow ambient air to flow into the chamber at a desired rate, which in turn causes the oxidation reaction in the heat generating medium to generate a desired temperature on the skin. The bottom of the chamber and frame wall are also substantially impermeable to oxygen. Within the chamber the heat generating medium which generally comprises activated carbon, iron powder, salt, and water. Agents that improve air flow, such as fine wood powder may also be added. The ratio of components in this embodiment is very important in order for the heat generating medium to work properly. For example, a typical ratio of approximately 5:16:3:2:6 of activated carbon: iron powder: fine wood powder: sodium chloride: and water (all weights) makes a reasonably good heat generating medium.

The heating patch which uses an oxidation reaction to generate heat needs to be stored in an air-tight container. When the integrated patch is removed from the container, oxygen in the ambient air flows into the shallow chamber, initiating a heat generating oxidation reaction in the heat generating medium. The amount of heat generated per unit of time is controlled by the rate of the oxygen flow into the heat generating medium through the cover. Less than the entire number and size of holes on the cover can be utilized to further control the amount of heat generated per unit of time.

Effectively, combining a heating patch as a temperature control component with a dermal drug delivery component such as briefly described above, results in an integrated dermal drug delivery system patch.

The integrated heating patch design allows a person or care giver to more conveniently apply controlled heat for the purpose of more effective transdermal drug delivery. Additionally, the integrated heating patch design helps to prevent the misuse or improper use of controlled heat with transdermal drug delivery. The integrated patch provides for a more uniform heating of the associated drug formulation. When a patient uses a drug delivery system with a separate temperature control element, it is possible that improper placement of the temperature control element by the user or unintended displacement of the heating element may result in uneven heating of the drug formulation. A separate temperature control element requires the patient or care giver to determine which kind of element to use, when to initiate heating, when to terminate heating, what temperature range is appropriate, and where and how to direct or attach the heat from the separate temperature control element. The actual handling may vary from patient to patient and treatment to treatment. A patient or care giver could easily make the wrong decision concerning the issues listed above and thus misapply the separate heating element. Improper use of the temperature control element may yield improper drug dosage. The integrated heat component and drug delivery component help to reduce or eliminate the potential for misuse of a separate temperature control element.

A preferred embodiment of the integrated heating patch comprises a tray made of a material that is a good barrier to volatile liquid, especially water, and alcohol but not necessarily a good barrier to oxygen. The tray defines a shallow reservoir capable of accommodating both a drug formulation and drug formulation applicator. The drug formulation adheres to the drug formulation applicator. The drug formulation applicator is secured to a film which is a good barrier to volatile liquids. The film can be heat sealed to the edge of the tray to form a closed compartment defined by the reservoir within the tray and the film. The drug formulation resides within the compartment. Since both the tray and lid to the compartment act as barriers to solvents, when the compartment is sealed tight, it prevents the transfer of substance(s) between the drug formulation and the outside environment.

On top of the film is an adhesive tape that has an area slightly larger than the film. The adhesive side of the adhesive tape faces the film and the edges of the tape extend out beyond the edges of the film. The portion of the adhesive tape which extend beyond the edges of the film is used to secure the integrated patch to the skin of the user. The heating patch is secured on top of the adhesive tape, and is centered on the adhesive tape. It is desirable that the inside area of the heating patch containing the heat generating medium be substantially the same or slightly larger than the area of the drug formulation applicator, to provide for effective heating. In other words, when the heating patch is secured to the film barrier and drug applicator, the heat generating element should be present directly above any areas of drug formulation so that substantially all of the drug formulation (which is also the barrier film) is evenly and uniformly heated.

In the preferred embodiment, the outer-most edges of the lidding film are not sealed onto the tray, and an adhesive tape is placed on top of the lidding film with the adhesive side adhered to the lidding film. The size of the adhesive tape may be similar to that of the lidding film, or preferably, slightly larger than the lidding film. If the adhesive tape is slightly larger than the lidding film, the portion of the adhesive tape that extends beyond the edges of the lidding film is rested on the tray. When a patient is removing the tape from the tray for use, the adhesive tape can be peeled from the tray at one end. The portion of the lidding film that is not sealed onto the tray (but is adhered to the adhesive tape) comes up with the adhesive tape. As the peeling continues, the entire lidding film, and the drug formulation attached to it, comes up with the adhesive tape. The adhesive tape, with the heating patch on the upper side and the drug formulation in the lower side, is then used to affix the integrated patch onto the skin. The tray may be indented at the end(s) to facilitate the start of the peeling.

The integrated patch is sealed in an air-tight container. The formulation is completely sealed in the space between the tray and the barrier film, so no exchange of substances between the formulation and the heat generating medium or the outside environment may take place. The heat generating medium is further sealed by the air-tight container so it is completely contained in the space inside the air-tight container. Thus the drug formulation is completely isolated from both the temperature control component and the outside environment. The temperature control component is isolated from the drug formulation. When the integrated patch is sealed into an air-tight container, the temperature control component is also isolated from the outside environment. Thus, the heating patch can be integrated with the drug delivery component and can be stored together in an air tight compartment such as a pouch made of film which is a good barrier to both air and moisture.

In one embodiment of the integrated DDDS patch, the barrier for preventing undesired substance transfer between the drug delivery system component and the temperature control component may comprise one or more chambers or compartments in which the drug delivery component and temperature control component are isolated while remaining structurally integrated. The chambers may be impermeable substances as required by the specific drug formulation and temperature control component. Similarly, means for preventing transfer of substances between the drug delivery and temperature control components with the external environment may comprise a chamber or pouch in which the integrated heating patch is stored. The chamber or pouch may be impermeable to moisture, oxygen, light or other environmental factors as necessary.

In another embodiment of the integrated heating patch, means for preventing undesired heat loss is provided. Means for preventing undesired heat loss includes insulating materials used in the drug delivery and temperature control components. Other means for preventing undesired heat loss include using adhesives and other means for securing and sealing the integrated DDDS patch to the skin of the user so that heat does not escape through unsecured edges or corners of the drug delivery component and temperature control component, as well as customized shaping or molding of the integrated heating patch to more appropriately fit a specific part of the user's body.

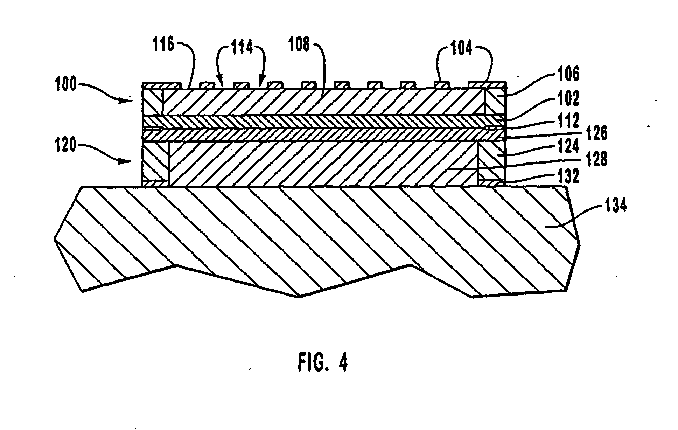

In one embodiment of the present invention, a means for preventing undesired heat loss is provided. In some instances it can be difficult to secure a corner of a patch to a user's skin. Figure 4 shows an integrated heating patch having a substantially oval shape. The oval shape does not have corners, as would a rectangular or square shaped patch. Thus the oval shape facilitates the prevention of heat loss through unsecured corners by eliminating corners which may be difficult to secure and result in undesired heat loss.

Another embodiment of the present invention provides a foam cover for the heat generating component. The foam tape cover has insulative properties which help to minimize heat loss through the cover and which help to prevent varying ambient temperatures from adversely affecting the heat generated by the heating, temperature control component. Moreover, an insulative cover capable of insulating the exposed surfaces of the integrated heating patch is also contemplated.

It is often necessary for the heat generating medium and the drug formulation to be entirely sealed from each other and from the external environment during storage and/or use. It is also desirable to provide convenient application and use of both components despite the sophisticated sealing necessary to preserve the drug formulation and heat generating medium. The novel configurations in this invention provide both satisfactory separation of the components during storage and/or use, and convenience in application and use.

The temperature control component and the drug delivery component of the present invention are preferably isolated. The isolated drug delivery component and the isolated temperature control component are disposed to prevent or avoid undesired interaction with the environment and with other components of the device. For example, the isolated temperature control component can be an exothermic medium enclosed in a substantial air-tight environment having a barrier which prevents undesired substance transfer among the heat generating medium in the temperature control component, the environment and the drug delivery component. Similarly, the isolated drug delivery component may be enclosed in a substantially air-tight compartment and may have a barrier to prevent any undesired substance transfer or exchange among the outside environment, the temperature control component and the drug delivery component. Isolation requirements for each component may differ depending upon the heat generating medium and the drug formulation being used.

Without careful designing, attempts to combine heat produced by exothermic oxidation reactions and transdermal drug delivery may result in an inoperative or ineffective combination. Some are rendered inoperative or ineffective because the components are not properly and conveniently isolated. Substances from the drug formulation may be lost to and/or foul the heat generating oxidation reaction elements through vapor absorption. During storage, the loss of substance(s) from the drug formulation may cause the drug formulation to function significantly differently than originally desired. Furthermore, substance(s) from the temperature control component may undesirably interact with the drug formulation rendering it less effective or ineffective. Other combinations are difficult or impractical to produce and use.

### BRIEF DESCRIPTION OF THE DRAWINGS

While the specification concludes with claims particularly pointing out and distinctly claiming that which is regarded as the present invention, the advantages of this invention may be more readily ascertained from the following description of the invention, when read in conjunction with the accompanying drawings in which:
FIG. 1 is a side cross-sectional view of an embodiment of a temperature control apparatus according to the present invention;
FIG. 2 is a side cross-sectional view of another embodiment of a temperature control apparatus according to the present invention;
FIG. 3 is a side cross-sectional view of an embodiment of a dermal drug delivery system according to the present invention;
FIG. 4 is a side cross-sectional view of the temperature control apparatus of FIG. 2 in conjunction with the dermal drug delivery system of FIG. 3 according to the present invention;
FIG. 5 is a graph of time versus temperature for a temperature control apparatus according to the present invention;
FIG. 6 is a graph of the mean fentanyl concentration of nine volunteers verse time for a four hour contact of a fentanyl containing DDDS with heating and without heating according to the present invention;
FIG. 7 is a graph of time versus temperature for a temperature control apparatus according to the present invention;
FIG. 8 is a side cross-sectional view of another embodiment of a temperature control apparatus according to the present invention;
FIG. 9 is a side cross-sectional view of another embodiment of a dermal drug delivery system according to the present invention;
FIG. 10 is a side cross-sectional view of the temperature control apparatus of FIG. 8 in conjunction with the dermal drug delivery system of FIG. 9 according to the present invention;
FIG. 11 is a side cross-sectional view of still another embodiment of a dermal drug delivery system according to the present invention;
FIG. 12 is a side cross-sectional view of the temperature control apparatus of FIG. 8 in conjunction with the dermal drug delivery system of FIG. 11 according to the present invention;
FIG. 13 is a side cross-sectional view of yet another embodiment of a temperature control apparatus having three cover layers over an oxygen activated temperature regulating mechanism chambers according to the present invention;
FIG. 14 is a side cross-sectional view of the temperature control apparatus of FIG. 13 having a first cover layer removed according to the present invention;
FIG. 15 is a top plan view of the temperature control apparatus of FIG. 14 along line 15-15 according to the present invention;
FIG. 16 is a side cross-sectional view of the temperature control apparatus of FIG. 14 having a second cover layer removed according to the present invention;
FIG. 17 is a top plan view of the temperature control apparatus of FIG. 16 along line 17-17 according to the present invention;
FIG. 18 is a side cross-sectional view of the temperature control apparatus of FIG. 16 having a third cover layer removed according to the present invention;
FIG. 19 is a top plan view of the temperature control apparatus of FIG. 18 along line 19-19 according to the present invention;
FIG. 20 is a side cross-sectional view of another embodiment of a dermal drug delivery system having a rate limiting membrane according to the present invention;
FIG. 21 is a side cross-sectional view of an electric temperature control mechanism according to the present invention;
FIG. 22 is a side cross-sectional view of a temperature control apparatus comprising a flexible bag filled with a supercooled liquid according to the present invention;
FIG. 23 is a side cross-sectional view of a temperature control apparatus applied directly to a patient's skin according to the present invention;
FIG. 24 is a side cross-sectional view an insulative material over a DDDS and injected or depot drug sites for minimizing temperature variation and/or increasing the temperature of the DDDS and the skin thereunder according to the present invention.
FIG. 25 shows a perspective view of a layered cut away of one embodiment of the integrated patch.
FIG. 26 shows an exploded view of the integrated patch in Figure 1.
FIG. 27 shows the patch in Figure 1 in cross-section as stored.
FIG. 28 shows a top, transparent view of the drug delivery component of one embodiment of the integrated patch.
FIG. 29 shows a cross section of a heating patch.

### DETAILED DESCRIPTION OF THE ILLUSTRATED EMBODIMENT

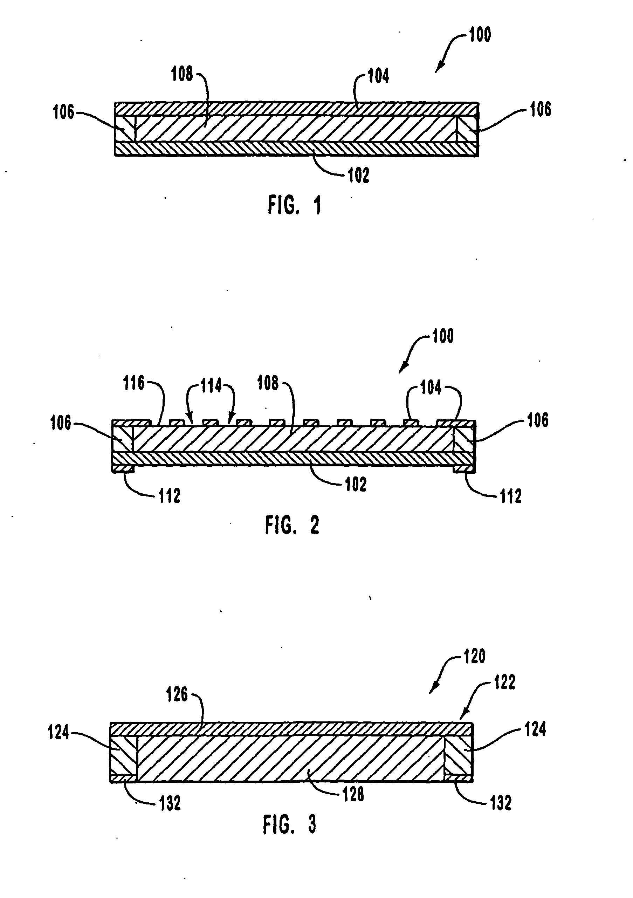

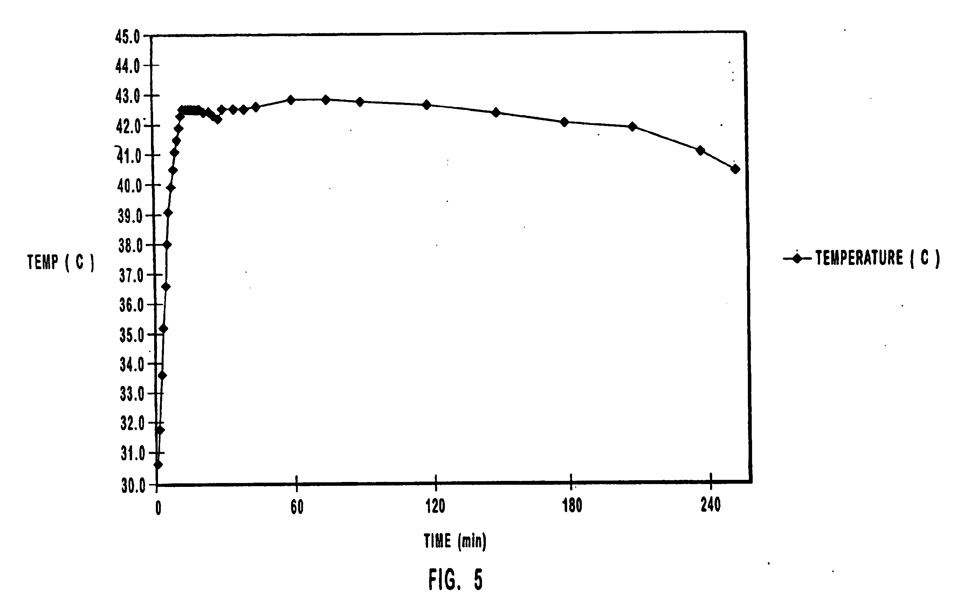

FIGs. 1-29 illustrate various views of temperature control or other apparatuses and dermal drug delivery systems. It should be understood that the figures presented in conjunction with this description are not meant to be illustrative of actual views of any particular apparatus, but are merely idealized representations which are employed to more clearly and fully depict the present invention than would otherwise be possible. Elements common between the figures retain the same numeric designations.

FIG. 1 illustrates a temperature control apparatus 100 of the present invention comprising a chamber defined by a bottom wall 102, a top wall 104, and side walls 106 wherein a temperature regulating mechanism 108 is disposed within the chamber. The temperature regulating mechanism 108 can include a heat generating oxidation reaction mechanism, electric heating unit, exothermic crystallization mechanism, endothermic crystallization mechanism, heating/cooling mechanism, cooling mechanism, or the like.

FIG. 2 illustrates a temperature control apparatus 100 comprising a temperature regulating mechanism 108 surrounded by a bottom wall 102, a top wall 104, and side walls 106. The bottom wall 102 is preferably a plastic material and the side walls 106 are preferably made of a flexible non-air permeable material, such as non-air permeable closed-cell foam material. A portion or all of the bottom wall 102 of the temperature control apparatus 100 includes an adhesive material 112 for attachment to a DDDS or to the skin of a patient. The temperature regulating mechanism 108 preferably comprises a composition of activated carbon, iron powder, sodium chloride and water in a proper ratio. Optionally, saw dust may be added to the composition to facilitate the airflow within the composition and/or provide "body" to the composition. The top wall 104 is preferably also a flexible non-air permeable material having holes 114 therethrough. An air permeable membrane 116 is, preferably, disposed between the top wall 104 and the temperature regulating mechanism 108 to regulate the amount of air reaching the temperature regulating mechanism 108 through the holes 114. The air permeable membrane 116 is preferably a porous film (such as No. 9711 microporous polyethylene film - CoTran™, 3M Corporation, Minneapolis, Minnesota, USA).

FIG. 3 illustrates a dermal drug delivery system 120 (hereinafter "DDDS 120") comprising a housing 122 made of a flexible material(s). The housing 122 preferably comprises side walls 124 and a top wall 126 with a drug formulation 128 disposed within the housing 122. Preferably, the bottom of the DDDS side walls 124 include an adhesive 132 to affix the DDDS 120 to the skin of a patient.

FIG. 4 illustrates the temperature control apparatus 100 of FIG. 2 attached to the DDDS 120 of FIG. 3. The DDDS 120 attached to a portion of the skin 134 of a patient. The area of the temperature regulating mechanism 108 is preferably slightly larger than that of the drug formulation 128. The temperature control apparatus 100 and the DDDS 120 are preferably stored in separated compartments of an air tight container (or in separate air tight containers).

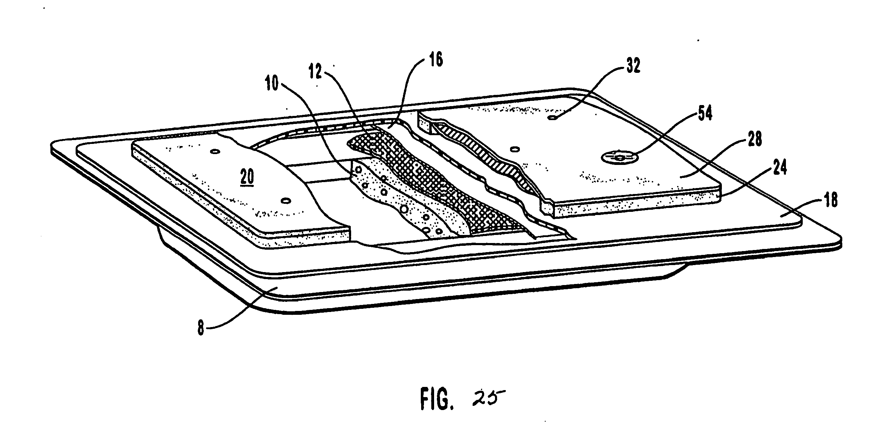

FIG. 25-27 illustrate various views of the integrated patch. The integrated patch 2 comprises a temperature control component 20 and a drug delivery component 4. The integrated patch 2 is stored within a foil pouch 6.

The drug delivery component 4 further comprises a tray 8 defining a drug formulation reservoir 40 and a drug applicator reservoir 42 substantially coextensive with drug formulation reservoir 40. Tray 8 has a rim 44 extending around its perimeter. Drug formulation 10 is disposed within formulation reservoir 40 of tray 8. Gauze 12 is disposed within applicator reservoir 42 and is in contact with the drug formulation disposed within formulation reservoir 40. Film barrier 16 is releasably secured to rim 44 of tray 8 and secured to gauze 12. Film barrier 16 secured to rim 44 of tray 8 defines a drug formulation compartment. Adhesive tape 18 is releasably secured to the rim 44 of tray 8 and is secured to the top of film barrier 16. Tape 18 is wider and longer than film 16, gauze 12 and drug formulation 10 such that adhesive tape 18 extends beyond the perimeters of film 16, gauze 12 and drug formulation 10. Adhesive on the bottom side of tape 18 is used to secure the integrated patch to the skin of the user and to secure tape 18 to tray 8.

The temperature control component 20 is further comprised of a patch reservoir 52. The patch reservoir may be defined by a medical tape base 22, a foam tape frame 24 and a foam tape cover 28. Heat generating medium 34 is disposed within patch reservoir 52. Cover 28 defines a plurality of holes 32. Holes 32 can be selectively covered and uncovered with an oxygen impermeable or air flow rate limiting hole cover 54. Hole cover 54 allows the permeability of cover 28 to be selectively adjusted by covering and uncovering some or all of holes 32 or by varying the duration that the holes are uncovered. Holes 32 may be covered with a membrane (not shown) having select air permeability.

In the preferred embodiment means for isolating the drug delivery component is a compartment defined by film barrier 16 and tray 8. Other means for isolating the component degradation of the drug delivery component may include alternative physical barriers.

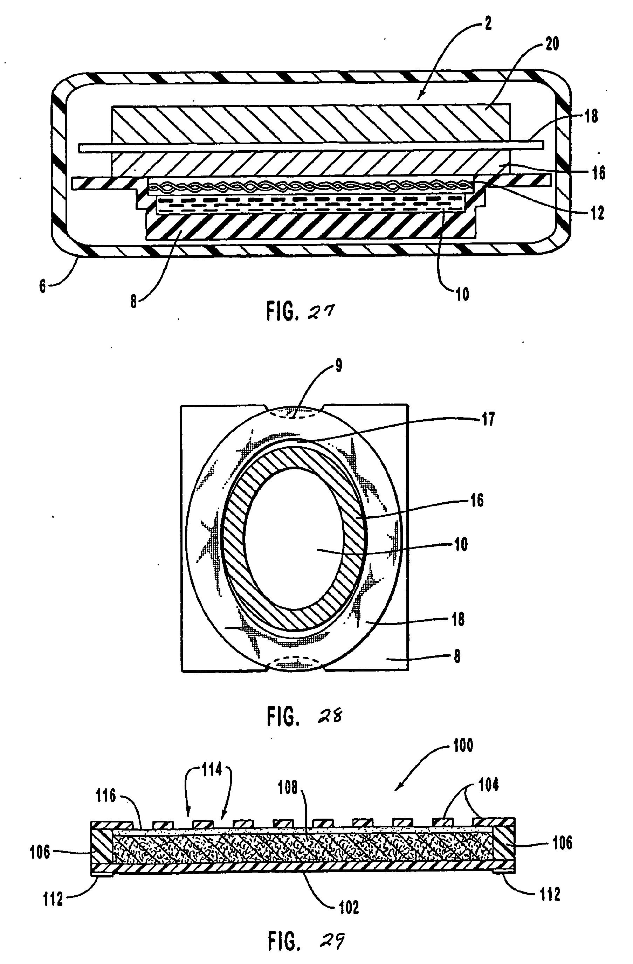

The preferred embodiment illustrated in Figure 27 shows a means for isolating the temperature control component comprising a foil pouch 6. Other means for isolating the component from external environmental factors include alternative physical barriers, for separating integrated patch components from the environment.

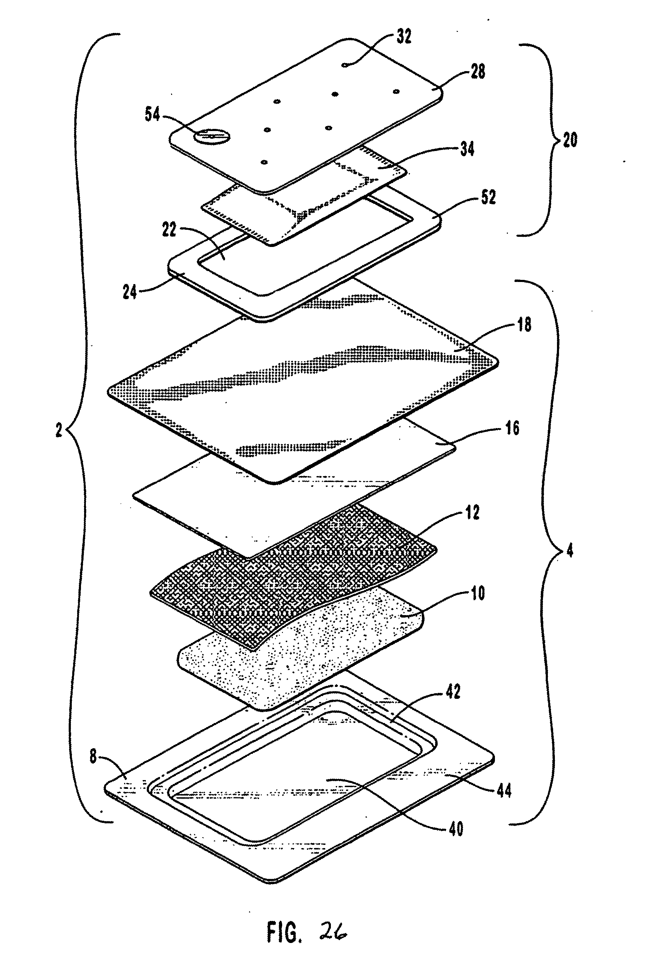

One embodiment shown in Figure 26 shows a temperature control component 20 comprising holes 32 defined in the non-permeable cover 28, holes 32 being selectively coverable and uncoverable. Other means for controlling the temperature are contemplated relative to the heat generating medium 34 in the temperature control component 20. Such alternative means would include electronic means for adjusting temperatures and alternative methods for controlling heat generated by exothermic reaction rates (i.e. ambient oxygen is let into the heat generating medium through the frame wall).

In the preferred embodiment illustrated in Figure 26 the temperature modification element is a heat generating medium 34, more specifically a medium capable of undergoing an exothermic oxidation reaction with oxygen. Other temperature modification elements are also contemplated such as an electronic heat generating element and alternative exothermic chemical reactions.

The relative positioning of the adhesive tape and the barrier film as well as the relative strength of the adhesive and the heat sealing are taken into account in order to provide easy removal of the patch from the tray without unintentionally disassembling the integrated patch 2. As shown in Figure 28, it is preferred that of the heat sealable film 16 have at least one end 17 remain unsealed to the tray, but adhered to the adhesive tape. This allows the heat sealable film 16 to be removed with the adhesive tape, rather than remaining secured to tray 8. In the embodiment shown in Figure 28, it has been found that it is suitable to terminate the heat sealing bond approximately 2 mm from the narrow end of the film 16.

In an actual experiment, the temperature control apparatus 100 comprised the side walls 106 defined by a 0,635cm (1/4 inch) thick rectangular foam tape (4 layers of No. 1779 0,159cm (1/16") white foam tape, 3M Corporation, Minneapolis, Minnesota, USA Corporation, Minneapolis, Minnesota, USA) with an outer dimension of about 5,72cm (2.25 inches) by 10,2cm (4 inches) with an opening therein inner dimension of about 4,45 (1.75) by 8,89 (3.5 cm (inches), the bottom wall 102 comprising rectangular medical tape (No. 1525L plastic medical tape, 3M Corporation, Minneapolis, Minnesota, USA Corporation, Minneapolis, Minnesota, USA) of a dimension of about 5,72cm (2.25 inches) by 10,2cm (4 inches) with a non-adhesive side attached to the bottom of the side walls 106, and a top wall 104 comprising a rectangular 1/32 inch thick foam tape (No. 9773 0,08cm (1/32") tan foam tape, 3M Corporation, Minneapolis, Minnesota, USA Corporation, Minneapolis, Minnesota, USA) with thirty-two holes 114 there through (diameters approximately 0,159cm (1/16 inch)). The side walls 106, the bottom wall 102, and the top wall 104 defined a chamber. The holes 114 of the top wall 104 were covered by an air permeable membrane 116 comprising a microporous membrane (no. 9711 CoTran™ membrane, 3M Corporation, Minneapolis, Minnesota, USA Corporation, Minneapolis, Minnesota, USA) disposed between the top wall 104 and the temperature regulating mechanism 108. The temperature regulating mechanism 108 disposed in the chamber comprised a mixture of activated carbon, iron powder, saw dust, sodium chloride and water in the weight ratio of approximately 5:21:3:2:6 weighing approximately 31 grams.

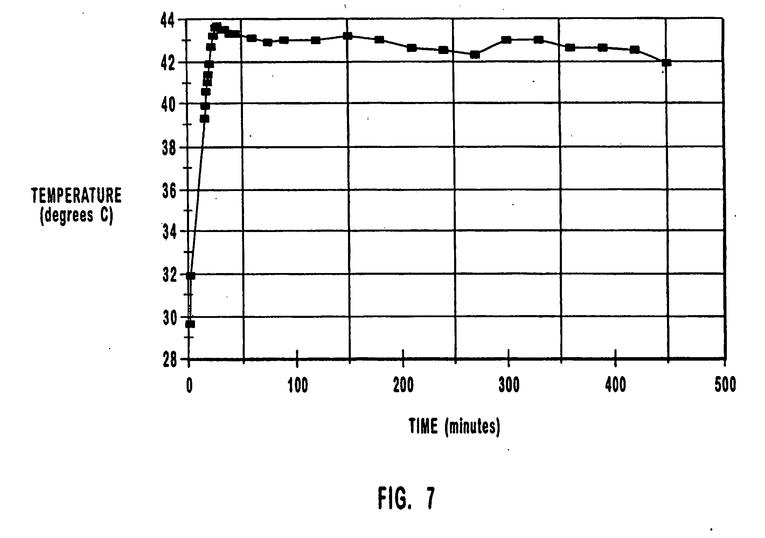

This temperature control apparatus 100 was tested on a volunteer's skin with a temperature probe placed between the temperature control apparatus 100 and the volunteer's skin to measure the temperature. The results of this temperature experiment are illustrated in FIG. 7 and Table D, which shows that the temperature control apparatus 100 was capable of keeping the skin temperature to a narrow, elevated range between about 41 and 44°C for extended periods of time (at least 840 minutes).

**TABLE D**

| Time (minutes) | Temperature (°C) |
|---|---|
| 0 | 31.9 |
| 1 | 32.2 |
| 2 | |
| 3 | 33.2 |
| 4 | 33.8 |
| 5 | 34.4 |
| 6 | 34.8 |
| 7 | 35.8 |
| 8 | 35.9 |
| 9 | 36.7 |
| 10 | 37.3 |
| 11 | 37.8 |
| 12 | 38.4 |
| 13 | 38.7 |
| 14 | 39.2 |
| 15 | 39.4 |
| 16 | 39.8 |
| 17 | 39.9 |
| 18 | 40.1 |
| 19 | 40.3 |
| 20 | 40.5 |
| 22 | 40.8 |
| 24 | 40.9 |
| 26 | 41 |
| 28 | 41.1 |
| 30 | 41.1 |
| 35 | 41 |
| 40 | 41 |
| 45 | 40.9 |
| 75 | 41.1 |
| 150 | 41.7 |
| 210 | 41.6 |
| 300 | 41.5 |
| 390 | 41.7 |
| 510 | 41.4 |
| 570 | 41.5 |
| 720 | 41.4 |
| 780 | 41.4 |
| 840 | 41.5 |

The results of the experiments noted above and the description of the heating patch offered herein indicate the heating patch provides unique advantages over the prior art and in some circumstances, over other methods of generating heat in a temperature control apparatus. The heating patch provides a safe means whereby the skin temperature can be elevated. Because the heat generating element is a controlled oxidation reaction between iron powder and oxygen, the chances of the heat generating element over heating and causing trauma to the skin are greatly reduced. Using a cover with selected air flow rate to limit the amount of oxygen reacting with the iron powder, the heat generated by the exothermic reaction can be maintained within a safe range of temperatures. Furthermore the heat generating element does not contain dangerous chemicals. Because the reactants are in powder form, they are less likely to leak if the compartment holding the reaction components is punctured, as would be the case if the components were primarily liquid.

The heating patch is also convenient to use. Because the heating patch is designed to have a selectively permeable barrier between the oxidation reactants and ambient oxygen, it is possible to vary and adjust the temperature being generated by the oxidation reaction within the compartment. This can be done by changing the number and/or size of the holes on the cover. One embodiment of the present invention provides air impermeable air hole coverings for the air holes on the cover. The user can selectively cover and uncover one or several holes to increase or decrease the rate of the exothermic reaction and thereby adjust and regulate the heat produced by the heat generating element. Alternatively, where the exposed surface area of an air permeable membrane significantly determines the rate that oxygen reacts with the heat generating element, a desired portion of the exposed surface area may be covered to regulate the temperature within the narrow range of temperatures available.

The duration of the heat can be varied by adjusting the time of the exposure of the reactants to oxygen and/or by adjusting the amount of the reactant that is placed within the compartment. The heating patch's capacity to be adjusted allows the heating patch to be used in a variety of ways to meet the needs of the patient or caregiver. Another convenient feature of the heating patch is its use of oxygen to activate the heat generating element. The heating patch is conveniently activated by simply exposing the heating patch to ambient oxygen, such as when the heating patch is taken out of its air tight pouch prior to application. The heating patch does not require an electric or other external power source to generate heat and thus is conveniently portable.

The heating patch is also unique in that it provides a low cost solution to the need for controlled heat in transdermal drug delivery. Manufacture of the heating patch is not unduly complex and the design and form of the heating patch make distribution and storage of the heating patch easy. The components for making the heating patch are readily obtained from established sources within the industry at a relatively low cost. The heating patch is also environmentally friendly.

Another novel feature of this invention is providing thermal insulation to the heat generating devices. As shown in Figure 1, one embodiment of the present invention insulates the heat generating medium from ambient air in all directions except the surface used for passing the heat to the object being heated (i.e. a transdermal drug patch, a skin area under the heating device). Thermal insulation 6 around the heat generating medium 8 in the device 4 helps to isolate the device 6 from the outside environment and allows better heat accumulation in the heat generating medium 8. That means, compared with a device without such thermal insulation, the same heating temperature can be achieved with lower heat generation rate. The present invention allows a given amount of heat generating medium to last longer, which is very desirable for devices that need to be compact. This may also minimize the potential for risk of burning the skin if the heating device is directly applied on the skin by allowing the heat generating medium to be effective while generating a slightly lower heat temperature.

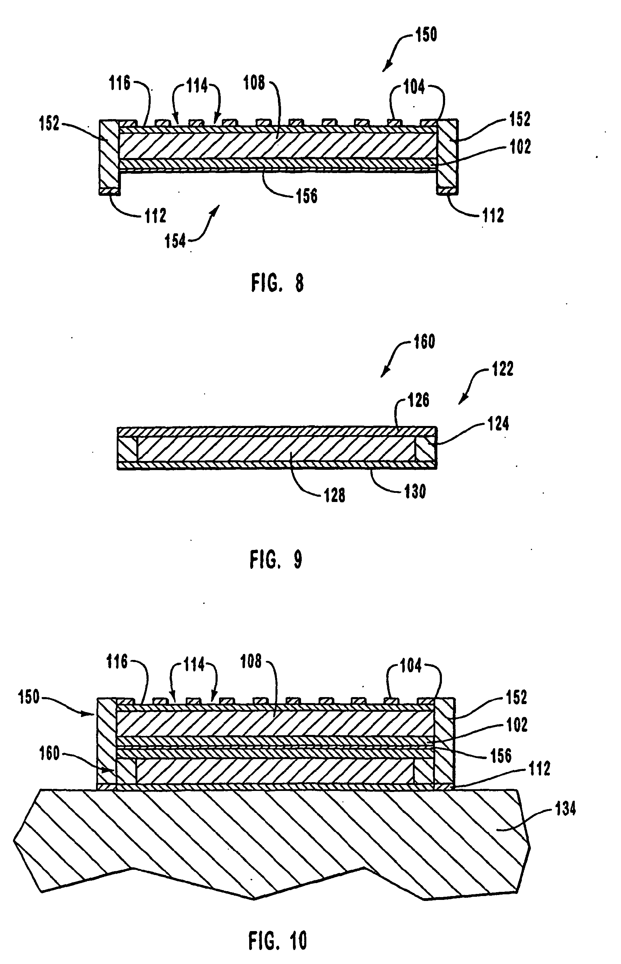

FIG. 8 illustrates another embodiment of a temperature control apparatus 150 comprising a temperature regulating mechanism 108 surrounded by a bottom wall 102, a top wall 104, and side walls 152. The side walls 152 extend a distance below the bottom wall 102 to define a cavity 154. The bottom wall 102 is preferably made of plastic tape material and the side walls 152 are preferably made of a flexible non-air permeable material, such as non-air permeable closed-cell foam material. A portion of the bottom of the temperature control apparatus 150 includes an adhesive material 112 on the bottom of the side walls 152 and, preferably, includes a second adhesive material 156 in the bottom of the bottom wall 102, wherein the second adhesive material 156 is preferably less adhesive than the adhesive material 112. Again, the temperature regulating mechanism 108 preferably comprises a composition of activated carbon, iron powder, sodium chloride, water, and, optionally, saw dust. The top wall 104 is preferably also a flexible non-air permeable material having holes 114 therethrough. An air permeable membrane 116 is disposed between the top wall 104 and the temperature regulating mechanism 108 to regulate the amount of air reaching the temperature regulating mechanism 108 through the holes 114.

FIG. 9 illustrates a DDDS 160 comprising a housing made 122 of flexible materials. The housing 122 preferably comprises side walls 124 and a top wall 126 with a drug formulation 128 disposed within the housing 122, and may include a membrane 130 which may be a rate-limiting membrane.

FIG. 10 illustrates the temperature control apparatus 150 of FIG. 8 attached to the DDDS 160 of FIG. 9. The DDDS 160 is placed on (or attached with an adhesive, not shown) a portion of the skin 134 of a patient and the temperature control apparatus 150 is placed over the DDDS 160, such that the DDDS 160 resides within the cavity 154 (see FIG. 8). The adhesive material 112 attaches to the skin 134 and holds the temperature control apparatus 150 in place. If the DDDS 160 is not attached to the skin 134, the temperature control apparatus 150 holds the DDDS 160 in place. Preferably, the DDDS 160 is attached to the skin 134 with an adhesive material (not shown) with the temperature control apparatus 150 placed over the DDDS 160. The temperature control apparatus 150 is attached to the skin 134 with the adhesive material 112 and the second adhesive material 156 (less adhesive than any attachment adhesive (not shown) between the DDDS 160 and the skin 134 and less adhesive than the adhesive material 112 between the temperature control apparatus 150 and the skin 134) attaches the temperature control apparatus 150 to the DDDS 160. Such an arrangement results in secure adhesion of the temperature control apparatus 150 and the DDDS 160 to the skin 134, yet allows for the removal of the temperature control apparatus 150 without removing the DDDS 160.

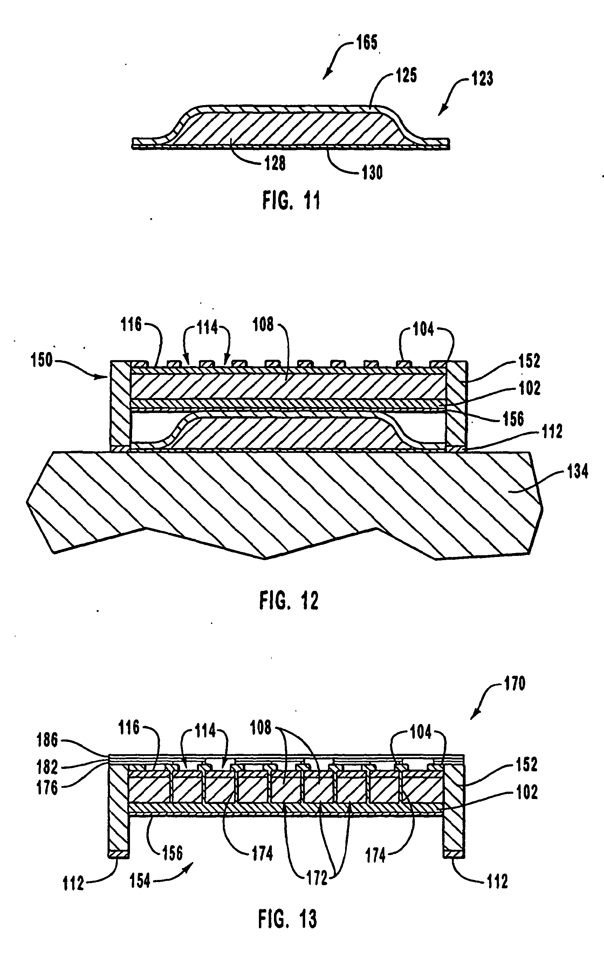

FIG. 11 illustrates an alternate DDDS 165 comprising a housing 123 made of flexible material(s). The housing 123 preferably comprises top wall 125 and a membrane 103, which may be a rate-limiting membrane, with a drug formulation 128 disposed within the housing 123. FIG. 12 illustrates the temperature control apparatus 150 of FIG. 8 attached to the DDDS 165 of FIG. 11, similar that described for FIG. 10.

### Example 1

Venous blood samples for serum testosterone concentration were obtained prior to the patch application (zero hours), and at 2, 4, 6, 8, 10 and 12 hours following patch application. After completing the blood draw at 12 hours, the patch system was removed. The Androderm® testosterone transdermal system which was selected was a 5 mg system having a total contact surface area of 24 cm² with a 15 cm² drug reservoir containing 24.3 mg testosterone USP dissolved in an alcohol-based gel. Serum testosterone concentrations were determined using a radioimmunoassay. Table B illustrates the serum testosterone concentrations at 0, 2, 4, 6, 8, and 10 hours. It was found that the administration of testosterone using an androgen testosterone transdermal system with a controlled heat aided drug delivery patch produced significantly higher testosterone concentrations in the subject's blood than using an Androderm® testosterone transdermal system patch alone.

**Table B**

| **Serum Testosterone Concentrations** | | |
|---|---|---|
| Time (hour) | Testosterone Concentration (ng/DL) Androderm® Patch | Testosterone Concentration (ng/DL) Androderm® + CHADD Patch |
| 0 | 340 | 325 |
| 2 | 456 | 722 |
| 4 | 402 | 961 |
| 6 | 567 | 1020 |
| 8 | 339 | 558 |
| 10 | 463 | 647 |

In a pilot study designed to determine if heat-generated from a control heated drug delivery system would significantly increase the absorption of testosterone, one adult human volunteer received an Androderm® 5 mg patch for 12 hours in the first treatment arm and an Androderm® 5 mg patch plus a CHADD controlled heat activated dermal delivery patch for 12 hours in the second treatment arm. A period of three days separated each treatment arm. Androderm® patches were produced by TheraTech, Inc., Salt Lake City, Utah, USA. The CHADD patch was manufactured by ZARS, Inc., and was similar in function and design to the heating patch used to generate the data of Table D (below). It was composed of five components; a foam tape cover, microporous membrane, a heat-generating medium, a foam tape reservoir, into which the heat-generating medium was placed, and a bottom adhesive tape layer. Except for the heat-generating medium, 3M Corporation, Minneapolis, Minnesota, USA drug delivery systems manufactures all the materials used in the CHADD patch components.

Thus, it is believed that the increased temperature increases the skin permeability (compared with an ATTS 120 without such a heating mechanism), which results in the testosterone entering the patient's systemic circulation faster. This should result in serum testosterone concentrations reaching desired therapeutic levels more quickly. The heating is also believed to increase the body fluid circulation and blood vessel wall permeability in the sub-skin tissues, and cause testosterone to spend less time in the sub-skin depot site. As a result, the patient receives the androgen compound more quickly and may receive improved treatment (in a situation where ATTS 120 without heating does not deliver a sufficient amount of testosterone.).

It is understood that the desired increase in androgen concentration in the systemic circulation may be an increase in the concentration of the androgen delivered by the delivery system, and/or a derivative of the androgen delivered by the delivery system and/or a different androgen. For example, testosterone enanthate may be delivered by a delivery system in order to increase the concentration of testosterone in systemic circulation. Thus, the delivery system androgen (in this case testosterone enanthate) facilitates an increase in the concentration of the target androgen (testosterone) in the systemic circulation.
1. In a second study designed to determine if heat generated from a control heated drug delivery system would significantly increase the absorption of testosterone, serum testosterone levels of six adult human volunteers were taken over a twelve hour period. Then the volunteers received an Androderm® 5 mg patch for 12 hours in a first treatment arm. Later, the volunteers received an Androderm® 5 mg patch plus a CHADD controlled heat activated dermal delivery patch for 12 hours in a second treatment arm. A period of three days separated each treatment arm. The Androderm® patches were produced by TheraTech, Inc., Salt Lake City, Utah, USA. The CHADD patches was manufactured by ZARS, Inc., and were similar in function and design to the heating patch used to generate the data of Table D (below). It was composed of five components; a foam tape cover, microporous membrane, a heat-generating medium, a foam tape reservoir, into which the heat-generating medium was placed, and a bottom adhesive tape layer. Except for the heat-generating medium, 3M Corporation, Minneapolis, Minnesota, USA drug delivery systems manufactures all the materials used in the CHADD patch components. Gauze tape was placed between the CHADD patch and Androderm patch to facilitate the removal of the CHADD patch after it was used.

Venous blood samples for serum testosterone concentration were obtained in the non-treatment arm at 0, 1, 2, 4, 6, 8, 10 and 12 hours to establish base line data. Venous blood samples for serum testosterone concentration were obtained prior to the patch application (zero hours), and at 1, 2, 4, 6, 8, 10 and 12 hours following patch application. After completing the blood draw at 12 hours, the patch system was removed. The Androderm® testosterone transdermal system which was selected was a 5 mg system having a total contact surface area of 24 cm² with a 15 cm² drug reservoir containing 24.3 mg testosterone USP dissolved in an alcohol-based gel. Serum testosterone concentrations were determined using a radioimmunoassay. Table B-1 illustrates the serum testosterone concentrations at 0, 1, 2, 4, 6, 8, 10, and 12 hours. As with the pilot study, this study showed that the administration of testosterone using an androgen testosterone transdermal system with a controlled heat aided drug delivery patch produced significantly higher testosterone concentrations in the subject's blood than using an Androderm® testosterone transdermal system patch alone.

**Table B-1**

| CHADD-Androderm study 5/99 | | | | | |
|---|---|---|---|---|---|
| Subject #1 | Serum Testosterone conc. (ng/DL) | | | | |
| Time (hr) | Natural baseline | Androderm alone | Androderm +CHADD | Andro-base | Andro+CH ADD-base |
| 0 | 500 | 515 | 354 | 15 | -146 |
| 1 | 425 | 442 | 566 | 17 | 141 |
| 2 | 464 | 429 | 626 | -35 | 162 |
| 4 | 542 | 687 | 967 | 145 | 425 |
| 6 | 567 | 470 | 706 | -97 | 139 |
| 8 | 353 | 498 | 735 | 145 | 382 |
| 10 | 315 | 506 | 742 | 191 | 427 |
| 12 | 388 | 614 | 727 | 226 | 339 |

| Subject #2 | Serum testosterone conc. (ng/DL) | | | | |
|---|---|---|---|---|---|
| Time (hr) | Natural baseline | Androderm alone | Androderm +CHADD | Andro-base | Andro+CH ADD-base |
| 0 | 299 | 287 | 314 | -12 | 15 |
| 1 | 357 | 314 | 400 | -43 | 43 |
| 2 | 335 | 321 | 544 | -14 | 209 . |
| 4 | 387 | 459 | 1020 | 72 | 633 |
| 6 | 350 | 561 | 641 | 211 | 291 |
| 8 | 397 | 509 | 555 | 112 | 158 |
| 10 | 408 | 519 | 536 | 111 | 128 |
| 12 | 335 | 523 | 597 | 188 | 262 |
| | | | | | |

| Subject #3 | Serum testosterone conc. (ng/DL) | | | | |
|---|---|---|---|---|---|
| Time (hr) | Natural baseline | Androderm alone | Androderm +CHADD | Andro-base | Andro+CH ADD-base |
| 0 | 360 | 394 | 414 | 34 | 54 |
| 1 | 319 | 413 | 530 | 94 | 211 |
| 2 | 271 | 455 | 666 | 184 | 395 |
| 4 | 362 | 604 | 878 | 242 | 516 |
| 6 | 389 | 677 | 590 | 288 | 201 |
| 8 | 234 | 551 | 597 | 317 | 363 |
| 10 | 295 | 588 | 542 | 293 | 247 |
| 12 | 327 | 635 | 658 | 308 | 331 |
| | | | | | |

| Subject #4 | Serum testosterone conc. (ng/DL) | | | | |
|---|---|---|---|---|---|
| Time (hr) | Natural baseline | Androderm alone | Androderm +CHADD | Andro-base | Andro+CH ADD-base |
| 0 | 279 | 272 | 246 | -7 | -33 |
| 1 | 285 | 241 | 441 | -44 | 156 |
| 2 | 243 | 341 | 514 | 98 | 271 |
| 4 | 214 | 322 | 592 | 108 | 378 |
| 6 | 261 | 394 | 586 | 133 | 325 |
| 8 | 268 | 380 | 587 | 112 | 319 |
| 10 | 240 | 393 | 456 | 153 | 216 |
| 12 | 205 | 363 | 477 | 158 | 272 |
| | | | | | |

| Subject #5 | Serum testosterone conc. (ng/DL) | | | | |
|---|---|---|---|---|---|
| Time (hr) | Natural baseline | Androderm alone | Androderm +CHADD | Andro-base | Andro+CH ADD-base |
| 0 | 373 | 266 | 434 | -107 | 61 |
| 1 | 370 | 409 | 567 | 39 | 197 |
| 2 | 419 | 502 | 847 | 83 | 428 |
| 4 | 314 | 667 | 1103 | 353 | 789 |
| 6 | 364 | 732 | 790 | 368 | 426 |
| 8 | 283 | 749 | 876 | 466 | 593 |
| 10 | 356 | 650 | 723 | 294 | 367 |
| 12 | 413 | 619 | 808 | 206 | 395 |
| | | | | | |

| Subject #6 | Serum testosterone conc. (ng/DL) | | | | |
|---|---|---|---|---|---|
| Time (hr) | Natural baseline | Androderm alone | Androderm +CHADD | Andro-base | Andro+CH ADD-base |
| 0 | 484 | 484 | 447 | 0 | -37 |
| 1 | 457 | 518 | 864 | 61 | 407 |
| 2 | 396 | 523 | 1057 | 127 | 661 |
| 4 | 385 | 742 | 857 | 357 | 472 |
| 6 | 395 | 608 | 1076 | 213 | 681 |
| 8 | 472 | 708 | 790 | 236 | 318 |
| 10 | 420 | 528 | 787 | 108 | 367 |
| 12 | 368 | 580 | 828 | 212 | 460 |
| | | | | | |
| Means | | | | | |

| Time (hr) | mean baseline | mean andro alone | mean andro +CHADD | mean andro-base | mean andro +CHADD-base |
|---|---|---|---|---|---|
| 0 | 382.5 | 369.67 | 368.17 | -12.83 | -14.33 |
| 1 | 368.83 | 389.5 | 561.33 | 20.67 | 192.5 |
| 2 | 354.67 | 428.5 | 709 | 73.83 | 354.33 |
| 4 | 367.33 | 580.17 | 902.83 | 212.83 | 535.5 |
| 6 | 387.67 | 573.67 | 731.5 | 186 | 343.83 |
| 8 | 334.5 | 565.83 | 690 | 231.33 | 355.5 |
| 10 | 339 | 530.67 | 631 | 191.67 | 292 |
| 12 | 339.33 | 555.67 | 682.5 | 216.3 | 343.17 |

### Example 2

Another example of using the embodiment of the present invention illustrated in FIGs. 8-12 for dermally administering nicotine for suppressing nicotine craving consists of a user placing a nicotine DDDS 160, 165 on the skin 134. After a few hours, the user should obtain a steady state nicotine concentration in the bloodstream that is sufficient to suppress a "baseline" nicotine craving. When the user starts to have an episode of increased nicotine craving, the user puts the temperature control apparatus 150 on top of the DDDS 160, 165. The temperature control apparatus 150 preferably heats for at least 15 minutes before the exothermic reaction exhausts the temperature regulating mechanism 108. The heat increases the transport of nicotine across the skin, and increases the blood flow in the tissues under the DDDS 160, 165 which carries nicotine stored in the tissues under the DDDS 160, 165 into the systemic circulation at increased rates. As a result, the user gets a rapid increase in his blood nicotine concentration to treat the surge of the nicotine craving. After the heating, the nicotine absorption rates gradually come back to normal to deliver the steady state nicotine concentration in the bloodstream.

### Example 3

Another example of using the embodiment of the present invention illustrated in FIGs. 8-12 for dermally administering testosterone to increase and optimize the amount of drug delivered consists of a user placing the DDDS 160, 165, such as a once a day dermal testosterone patch, for example Androderm^{®} produced by TheraTech, Inc. of Salt Lake City, Utah, USA, on the skin 134. The DDDS 160, 165 is generally applied to the skin 134 at night, for example at 10 PM. However, if the user does not get a sufficient dosage of testosterone the next day, the user puts the temperature control apparatus 150 on top of the DDDS 160, 165. The increased temperature in the DDDS 160, 165, the skin 134 and tissues under the skin significantly increase the dermal absorption of testosterone. In addition, if the DDDS 160, 165 has permeation enhancer, such as glycerol monooleate, the heat should also make the enhancer permeate the skin faster, thus making it more effective. The ultimate result is that the user gets sufficient testosterone from the DDDS 160, 165. Furthermore, the user may also place the temperature control apparatus 150 on the DDDS 160, 165 in the morning to deliver more testosterone from morning to the evening when the user needs the higher dosage the most. The increased absorption of testosterone by the controlled heating may allow the reduction of a permeation enhancer concentration which is used in the DDDS 160, 165. In a testosterone DDDS, a permeation enhancer is usually necessary for delivering sufficient testosterone, however permeation enhancers may cause serious skin irritation, such as glycerol monooleate in Andmderm^{®}.

### Example 4

It is, of course, understood that the DDDS 160, 165 and the temperature control apparatus 150 can be with athletic injuries. For example, if a person injures an elbow in a sporting event or such, the user can apply a DDDS 160, 165 containing an analgesic, such a dexamethasone, wintergreen oil, or the like, wherein the DDDS 160, 165. The heat generated by the temperature control apparatus 150 drives more drug into the elbow and the increased the blood flow induced by the heat takes the drug deeper into the elbow.

### Example 5

Yet another example of using the embodiment of the present invention illustrated in FIGs. 8-12 comprises using the temperature control apparatus 150 for administering a drug when the diffusion coefficient of the active ingredients in the formulation 128 and/or permeability coefficient across a rate limiting membrane 130 is so low that it dominantly determines the overall absorption rate of the drug from the DDDS 160,165 into a patient's body. By way of example with the use of a DDDS 160, 165, the patient or care giver places the DDDS 160, 165 on the skin 134 of the patient. If after a time of wearing the DDDS 160,165, it is determined that for this particular patient and his conditions a higher concentration of the drug in the bloodstream is required to properly treat his condition, the temperature control apparatus 150 is placed on top of the DDDS 160, 165 to heat the DDDS 160, 165.

The increased temperature increases diffusion coefficient of the active ingredient in the formulation in the DDDS 160, 165 and increases the permeability coefficient across the rate limit membrane 130 in the DDDS 160, 165, and, thus, the overall rates at which the active ingredient enters the patient's body. This, in turn, increases the concentration of active ingredient in the bloodstream. As a result, the patient gets the increased and proper effect.

### Example 6

Still another example of using the embodiment of the present invention illustrated in FIGs. 8-12 comprises using the temperature control apparatus 150 for decreasing onset time of a drug from the DDDS 160, 165. By way of example, the patient or care giver places the DDDS 160, 165 on the skin 134 of the patient and places the temperature control apparatus 150 over the DDDS 160. Preferably, the temperature control apparatus 150 includes a sufficient amount of activated carbon, iron powder, sodium chloride, and water in the temperature regulating mechanism 108 to sustain an exothermic reaction for at least 4 hours.

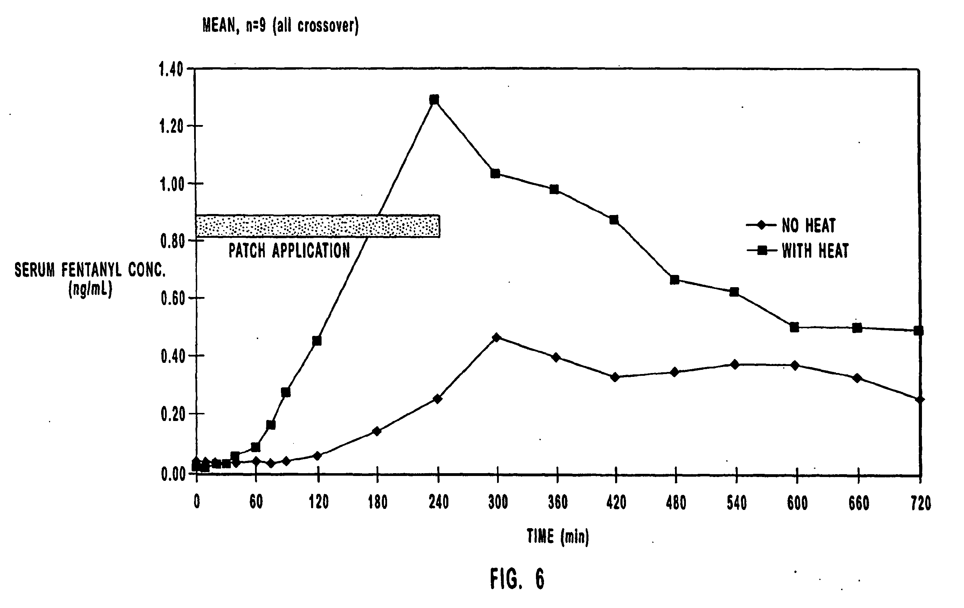

The heat from the temperature control apparatus 150 increases the temperature at a contact surface of the skin 134 and the DDDS 160, 165 to temperatures up to about 60°C, preferably a narrow temperature range between about 36°C and 46°C, most preferably between 37°C and 44°C, and maintains this temperature for a period of time (*i.e.*, approximately 4 hours). During this time, the heat increases the speed of the drug release from the DDDS 160, 165, the permeation rate across the skin 134, and the speed of blood circulation which carriers the drug into the systemic circulation faster. After the exothermic reaction ceases (approximately 4 hours), the drug absorption and concentration in the bloodstream begins to decrease from the elevated levels caused by the heat from the DDDS 160, 165 returns to normal (unheated) levels. The patient continues to wear the system for a total of between about 48 and 72 hours. Compared with a DDDS 160, 165 without the use of the temperature control apparatus 150, the drug begins to appear in the bloodstream significantly earlier to yield a shortened onset time and the drug concentrations in the bloodstream in the early hours of application are significantly higher than that produced by an unheated DDDS 160, 165. The therapeutic serum drug concentration varies from person to person. For example some people respond to levels above 0.2 ng/mL. Referring to FIG. 6, this 0.2 ng/mL concentration is achieved in about one-third the amount of time for a heated system than for a non-heated system (*i.e*., about 70 minutes as compared with about 210 minutes).

After a period of time when the exothermic reaction of temperature control apparatus 150 slowly stops generating heat, the drug concentration in the bloodstream starts to gradually approach the normal steady state drug concentrations in the bloodstream which would ultimately be seen with an unheated DDDS 160, 165, given a sufficient amount of time. As a result, the temperature control apparatus 150 significantly shortens the onset time of the drug in the patch without significantly altering its steady state delivery rates. Thus, the important advantage provided by this approach is that the onset time of a DDDS 160, 165 already in clinical use can be shortened without significantly altering its steady state delivery rates which are not only adequate, but also familiar to the caregivers and the patients.

### Example 7

Yet still another example of using the embodiment of the present invention illustrated in FIGs. 8-12 again comprises using the temperature control apparatus 150 for decreasing onset time of an analgesic material from the DDDS 160, 165. By way of example, a local anaesthetic, such as a eutectic mixture of lidocaine and tetracaine, can be administer with a DDDS 160, 165 to numb the skin 134 before a painful medical procedure. A faster onset and deeper numbing effect within a short time can be achieved by placing the temperature control apparatus 150 over the DDDS 160,165, wherein the temperature control apparatus 150 is capable of providing heating the skin to a narrow range between about 37°C and 41°C, preferably between 39°C and 40°C, for at least 30 minutes. The skin 134 should be numb in 30 minute or less, which is much shorter than that without heating. Depending on the original skin temperature, it is believed that such heating will reduce the onset time by about 60% of the onset time without heating.

### Example 8

Still another example of using the embodiment of the present invention illustrated in FIGs. 8-12 again comprises using the temperature control apparatus 150 for increasing the solubility of a drug from the DDDS 160, 165. By way of example, a formulation may be designed to contain a drug which has such low solubility in the formulation that a significant portion is in the form of undissolved particles, and the solubility increases with increasing the temperature of the formulation.

A patient places such a DDDS 160,165 on his skin. If the amount of the drug compound the patient receives from the DDDS 160, 165 is not sufficient, the patient places the temperature control apparatus 150 on or over the DDDS 160, 165. The heat generated in the temperature control apparatus 150 increases the temperature of the formulation in the DDDS 160, 165 and maintains the increased temperature for a significant part or substantially the entire length of the DDDS 160, 165 application. The increased temperature in the formulation increases the solubility of the drug compound in the formulation. Consequently, more drug compounds are dissolved in the formulation which gives higher driving force for the transdermal permeation of the drug compound. As a result, more of the drug compound enters the patient's body.

Although Examples 1-10 discuss the application of specific drugs, it is, of course, understood that the present invention is not limited to any particular drug(s). It is understood that a considerable variety of drugs classes and specific drugs may be used with the present invention. The drug classes can include without limitation androgen, estrogen, non-steroidal anti-inflammatory agents, anti-hypertensive agents, analgesic agents, anti-depressants, antibiotics, anti-cancer agents, local anesthetics, antiemetics, anti-infectants, contraceptives, anti-diabetic agents, steroids, anti-allergy agents, anti-migraine agents, agents for smoking cessation, and anti-obesity agents. Specific drugs can include without limitation nicotine, testosterone, estradiol, nitroglycerin, clonidine, dexamethasone, wintergreen oil, tetracaine, lidocaine, fentanyl, sufentanil, progestrone, insulin, Vitamin A, Vitamin C, Vitamin E, prilocaine, bupivacaine, sumatriptan, and dihydroergotamine.

### Example 9

Yet still another example of using the embodiment of the present invention illustrated in FIGs. 8-12 again comprises using the temperature control apparatus 150 for maintaining a stable temperature for the DDDS 160, 165. Certain drugs have relatively low therapeutic indices, meaning that the differences between the therapeutic dose and the dose which can cause serious and/or undesired side effects are small. Thus, dermal delivery of such drugs can be dangerous (over-dose) or ineffective (under-dose), especially for individuals whose skin are exposed to highly variable ambient temperatures, such as people working outdoors in extreme weather conditions. The variations in ambient temperature can cause variations in skin temperature which can significantly change the ultimate dermal absorption of the drugs. Covering a DDDS 160, 165 containing a low therapeutic indices drug with the temperature control apparatus 150 can regulate the skin temperature to a narrower range and reduce the variation in dermal drug absorption. Drugs and classes of drugs that may benefit from this method include, but are not limited to, drugs such as nicotine, nitroglycerin, clonidine, fentanyl, sufentanil, and insulin; and classes of drugs such as non-steroidal anti-inflammatory agents, anti-hypertensive agents, analgesic agents, anti-diabetic agents, and anti-migraine agents.

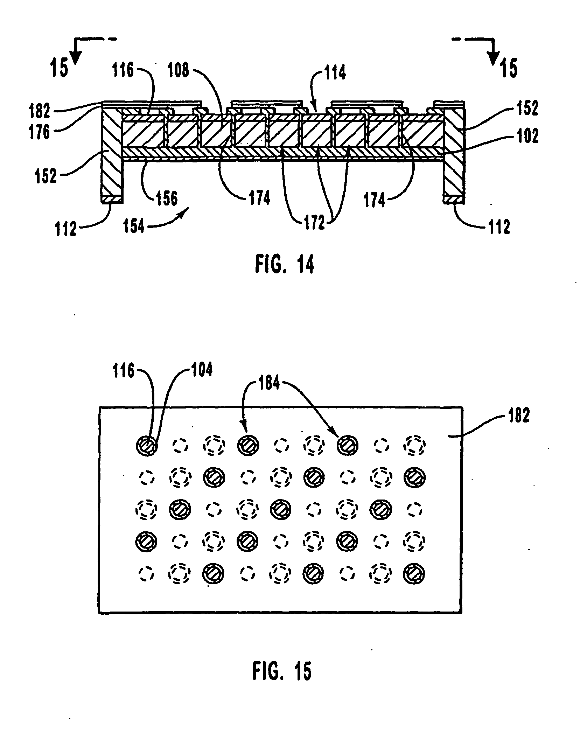

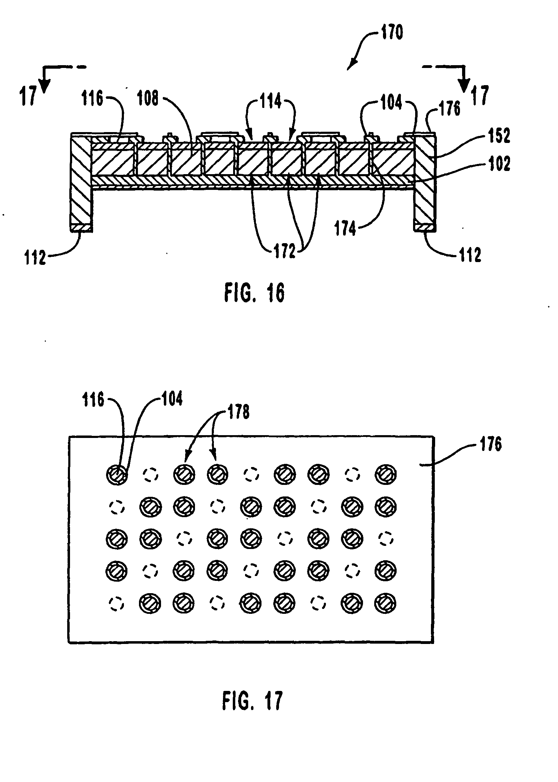

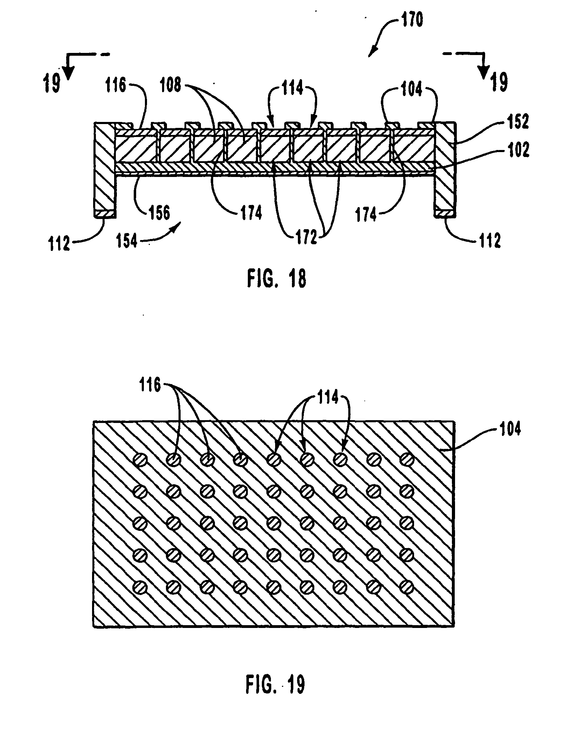

FIGs. 13-19 illustrates another embodiment of a temperature control apparatus 170. FIG. 13 illustrates the temperature control apparatus 170 which is similar to the embodiment of FIG. 8, but comprises a temperature regulating mechanism 108 which is made up of a plurality of chambers 172 separated by non-air permeable walls 174. The temperature regulating mechanism 108 is substantially surrounded by a bottom wall 102, a top wall 104, and side walls 152. Again, the temperature regulating mechanism 108 preferably comprises a composition of activated carbon, iron powder, sodium chloride, water, and, optionally, saw dust, which is disposed in each of the chambers 172. The top wall 104 is preferably also a flexible non-air permeable material having a plurality of holes 114 therethrough, preferably, a row of holes 114 for each chamber 172. An air permeable membrane 116 is disposed between the top wall 104 and the temperature regulating mechanism 108 to regulate the amount of air reaching the temperature regulating mechanism 108 through the holes 114. The top wall 104 can have at least one cover covering the plurality of holes 114 for the regulation of the air into the chambers 172. As illustrated in FIG. 13, three covers are layered on the top wall 104. A first cover layer 176 is affixed to the top wall 104 and has openings 178 (see FIG. 17) to expose 2 out of 3 holes 114. A second cover layer 182 is affixed to the first cover layer 176 and has opening 184 (see FIG. 15) to expose 1 out of 3 holes 114. A top cover 186, which has no openings, is affixed to the second cover layer 182. Thus, a patient has a various opinions on what percentage of chambers 172 to expose to ambient air. If the heat generated from one third of the chambers is required, the top cover 186 is removed, as shown in FIGs. 14 and 15. If the heat generated from two thirds of the chambers is required or if another additional heat is needed after the depletion of the first one-third of the temperature regulating mechanism 108, the top cover 186 and the second cover layer are removed, as shown in FIGs. 16 and 17. If the heat generated from all of the chambers is required or if another additional heat is needed after the depletion of the first and second one-third of the temperature regulating mechanism 108, the top cover 186, the second cover layer 182, and the first cover layer 176 are removed, as shown in FIGs. 18 and 19. It is, of course, understood that more or less cover layers can be used with any number of holes to results in any desired amounts of the temperature regulating mechanism 108 being activated.

Thus, by way of example a patient can have a number of choices in using the temperature control apparatus 170, such for the suppression of breakthrough pain. When the breakthrough pain occurs, the patent places the temperature control apparatus 170 over an analgesic material DDDS and can do any of the following:
1) Activate a particular number or percent of chambers 172 by removing the requisite covers depending on how much additional analgesic material is required to treat the breakthrough pain. The covers can be preferably replaced to stop the exothermic reaction when no more additional analgesic material is required.
2) Activate a particular number or percent of chambers 172, exhaust the heat generating capacity of those chambers 172, and then activate other (non-activated) chambers 172. This extends the heating duration of the temperature control apparatus 170. The duration of the total heating time is determined by the typical duration of the particular patient's breakthrough pain.
3) Activate enough chambers 172 to treat one episode of breakthrough pain, and leave the heating patch in place. When the next episode of breakthrough pain occurs, activate unused chambers 172.

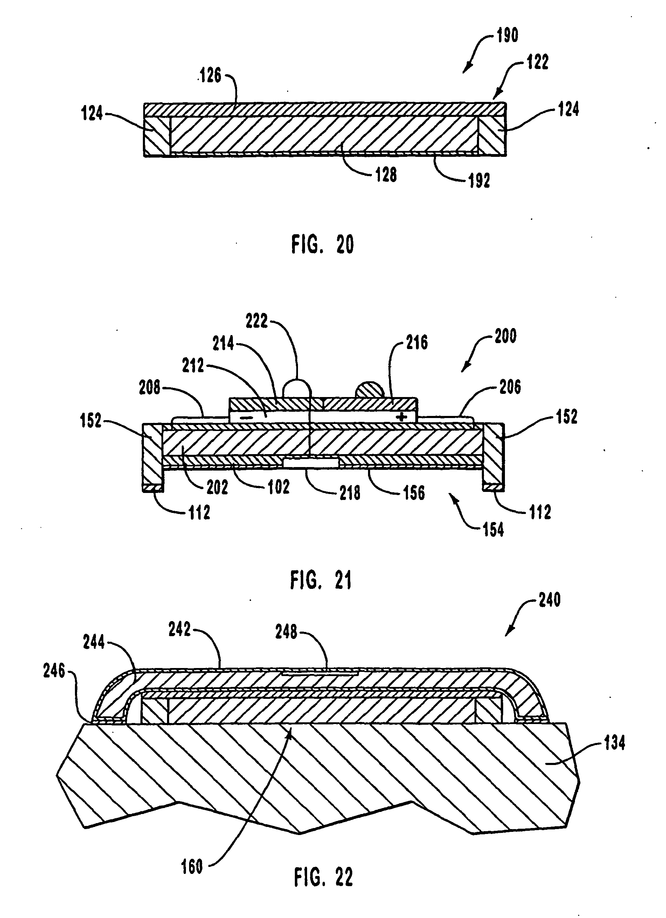

FIG. 20 illustrates a dermal drug delivery system 190 (hereinafter "DDDS 190") having a rate limiting membrane 192. The structure of DDDS 190 is similar to that of FIG. 3. However, the DDDS 190 includes a rate limiting membrane 192 which resides between the drug formulation 128 and the skin 134 of a patient.

Generally, the permeability of the drug in the drug formulation 128 through the rate limiting member 192 is significantly lower than the permeability of the drug in the drug formulation 128 into the skin of an average patient. Rate limiting membranes 192 are used to minimize the variation in overall permeation, and to regulate the amount of drug delivered to the patient so that overdosing does not occur. Another aspect of the present invention is the use of a temperature sensitive rate limiting membrane, such that the drug permeation rate through the rate limiting membrane increases significantly with increasing temperature. With such a DDDS 190, the above discussed temperature control mechanisms 100 (FIG. 1 & 2), 150 (FIG. 8), and 170 (FIG. 13) can be used to increase the drug delivery rate across the rate limiting membrane 192 to treat breakthrough pain, reduce onset time, increase steady state delivery rate, or other advantages discussed above.

The possible temperature control mechanisms are not limited to the exothermic reaction mixture of iron powder, activated carbon, salt, water, and sawdust, as discussed above. FIG. 21 illustrates an electric temperature control mechanism 200 comprising an electric heating element 202 surrounded by a bottom wall 102, a top wall 104, and side walls 152 (similar to FIG. 8). The side walls 152, preferably, extend a distance below the bottom wall 102 to define a cavity 154. It is, of course, understood that the electric heating element 202 does not have to have the side walls 152 forming a cavity 154.

The bottom wall 102 and the side walls 152 are preferably made of a flexible non-air permeable material, such as non-air permeable closed-cell foam material. A portion of the bottom of the temperature control apparatus 200 includes an adhesive material 112 on the bottom of the side walls 152 and, preferably, includes a second adhesive material 156 in the bottom of the bottom wall 102, wherein the second adhesive material 156 is preferably less adhesive than the adhesive material 112. The electric heating element 202 preferably comprises a flexible resistor plate that can generate heat when supplied with an electric current through traces 206, 208. The electric current is preferably supplied from a battery 212 attached to a control mechanism 214, and an electronic switch 216. The battery 212, the control mechanism 214, and the electronic switch 216 are preferably attached to the top surface of the top wall 104. The electric heating element 202 is activated by triggering the electronic switch 216 which begins the flow of electric current from the battery 212 to the electric heating element 202. A temperature sensor 218, such as a thermistor, is preferably attached to the bottom of the bottom wall 102 and sends a signal (corresponding to the temperature at the bottom of the bottom wall 102) through electric trace 222 to the control mechanism 214. The control mechanism 214 regulates the flow of current to the electric heating element 202, so that the electric heating element 202 quickly brings the temperature at a contact surface between the bottom wall 102 and a top of a DDDS (not shown) to a pre-determined level and maintains the temperature at that pre-determined level. The following features may be incorporated into the control mechanism 214: 1) a mechanism that allows a physician or care giver set the length of each heating period for each patient, which allows the physician to limit the heating, and hence the extra drug that the patient can get based on the conditions of the patient; 2) a mechanism that allows the physician or care giver to set the minimum time between the heating periods, and hence how often the patient can get the extra drug through increase heat; 3) a mechanism that allows the physician or care giver to set a pre-determined temperature; and/or 4) a mechanism that allows the physician or care giver to control the heating temperature profile, such as gradually increasing heating temperature or decreasing temperature over a pre-determined period of time. These features can potentially give simple DDDSs a variety of control options for the physician and/or the patient on the quantity and timing of the delivery of extra drug.

### Example 10

An example of using the embodiment of the present invention, such as illustrated in FIG. 21, includes using the temperature control mechanism 200 for decreasing onset time of a local anesthetic comprising approximately 14% tetracaine/lidocaine eutectic mixture by weight; 8.6% polyvinyl alcohol (PVA) by weight, 0.17% sodium hydroxide (NaOH) by weight, and the remainder water (H₂O). The local anesthetic, in the form of a thin patch, was placed on a volunteer's left forearm and the temperature control mechanism 200, set to maintain a 41°C temperature, was placed over the local anesthetic. The local anesthetic was also placed on a volunteer's right forearm (at a different time) and left at room temperature (about 24°C). The results are presented in Table D, wherein the effect of the local anesthetic was measure by a pain score when the skin is poked by a blunt object. The pain score is defined as follows:

| Score | Effect |
|---|---|
| 0 | No effect |
| 1 | Between no numbness and medium numb |
| 2 | Medium numb |
| 3 | almost completely numb |
| 4 | completely numb, but not deep |
| 5 | completely numb and deep |

**TABLE D**

| Time (minutes) | Pain Score with Heating | Pain Score w/o Heating |
|---|---|---|
| 15 | 4 | 2 |
| 20 | 5 | 3 |
| 25 | | 4 |
| 30 | | 5 |

Thus, it can be seen that heating reduced the onset time of complete and deep numbness by approximately 33%.

### Example 11

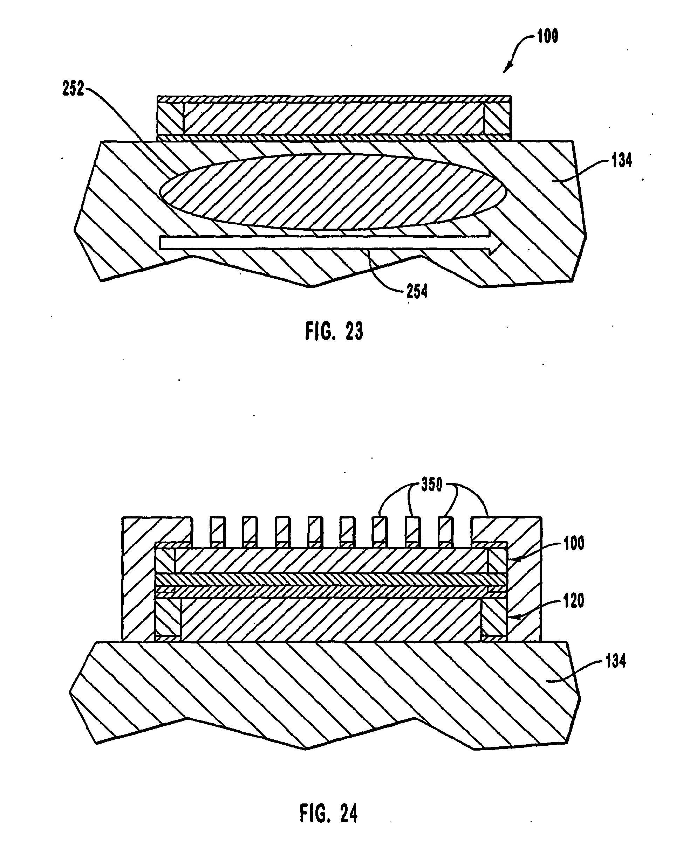

An example of using the embodiment of the present invention illustrated in FIGs. 23-24 comprises using a temperature control apparatus 300 which is capable of heating and cooling, such that the rate of absorption of a drug formulation in a DDDS can be increased or decreased, as needed.

For example, as shown in FIG. 23, if the level of the drug in the patient's system requires adjusting, the temperature control apparatus 300 is placed on a DDDS 160. Heating will result in an increase in drug absorption (as previously discussed) and cooling will reduce drug absorption to prevent overdose. FIG. 23 illustrates the temperature control apparatus 300 as a thermoelectric module which is be used for both heating or cooling. The temperature control apparatus 300 functions as a small heat pump, wherein a low voltage DC power source 304 provides a current in one direction 306 to a thermoelectric unit 310 which results in heating on a first side 308 (preferably a ceramic substrace) of the temperature control apparatus 300 and cooling on a second side 312 (preferably a finned dissipation structure) of the temperature control apparatus 300. If the current direction is reversed, the first side 308 will cool and the second side will heat.

The temperature control apparatus 300 may be control with a closed loop temperature controller 314, as shown in FIG. 24. The temperature controller 314 comprises a positive DC node 316 and a negative DC node 318 supplying circuit to a primary circuit 320. The primary circuit 320 delivers an electrical signal 322 through a voltage amplifier 324 and a power amplifier 326 to the thermoelectric unit 310. The primary circuit 320 further includes a temperature sensor 328 receiving a temperature signal 330 from the thermoelectric unit 310, and further includes a temperature adjustment mechanism 332, which adjusts the electrical signal 322.

A variety of drugs and drug classes can be utilized with such treatments. The drugs include, but are not limited to, nicotine, nitroglycerin, clonidine, dexamethasone, fentanyl, sufentanil, and insulin. The drug classes include, but are not limited to, androgen, non-steroidal anti-inflammatory agents, anti-hypertensive agents, analgesic agents, anti-depressants, anti-cancer agents, anti-diabetic agents, steroids, anti-migraine agents, anti-asthma agents, and agents for smoking cessation.

It is, of course, understood that the heating devices discussed above could be replaced by an infrared heating device with a feedback mechanism. All of the controls and variations in controls discussed above would apply to such an infrared heating device. The advantage of infrared radiation over simple heat is that the former, with proper wavelengths, penetrates deeper into a patient's skin.

Another aspect of the present invention is to use heat and other physical means, such as ultrasound, microwave, electric current, and vibration, to improve absorption of drugs from depot/storage sites. Such depot/storage sites may exist as a result of a drug administered from a dermal patch or a drug directly injected or implanted under the skin surface.

The kind of formulations that may respond to the physical inducing means discussed above are:

| | |
|---|---|
| Ultrasound: | particles containing drug formulation that can break down in size when treated with ultrasound. |
| Microwave: | drugs that have limited solubility in surrounding body fluid, but the solubility increases significantly with increasing temperature; and solid formulations whose erosion/degradation speed can be significantly increased by increasing flow/exchange of body fluid surrounding it. |
| Electricity: | drugs that exist in ionized form in the formulations and/or surrounding body fluid. |
| Vibration: | drugs that have limited solubility in body fluid; solid formulations whose erosion/degradation speed can be significantly increased by increasing flow/exchange of body fluid surrounding it. |

### Example 12

An example of storage site absorption using the embodiment of the present invention illustrated in FIGs. 1 and 2 consists of a patient or care giver introducing an extended release insulin into his skin by injection or other method such as ultrasound speed hitting (such as products similar to those developed by Powderject Pharmaceutical, United Kingdom). In the extended release insulin formulation, most of the insulin molecules are in crystalline form. After injection, insulin is released from the crystalline from slowly as the crystals slowly dissolve in the surrounding body fluid. This provides a baseline insulin release into the systemic circulation. However, the patient needs additional insulin above the baseline release to suppress sugar from meals. Thus, before each meal the patient places a temperature control apparatus 100, preferably designed to control heat for a pre-determined time (*i.e*., between about 15 and 60 minutes), onto the skin over the injection site where the injected extended release insulin formulation resides. The heat from the temperature control apparatus 100 increases flow of the blood and another body fluid in the tissues surrounding the extended insulin formulation, which increases the dissolution speed of the insulin and carries the insulin into the systemic circulation at higher rate. The heating duration of the temperature control device 100 is, preferably, designed to last just long enough to release the adequate amount of extra insulin to deal with the sugar from the meal. Thus, the patient receives proper insulin absorption adjustment from the extended release formulation, and does not have to make a choice between taking additional insulin shots before meals or suffer the physiological consequences caused by high blood sugar from the meals.

### Example 13

Another example of storage site absorption using the embodiment of the present invention illustrated in FIGs. 1 and 2 consists of a patient or care giver injecting a drug mixed in controlled release particles under the skin surface. The drug is incorporated into a controlled release drug delivery system (such as Atrigel™ by Atrix Laboratories, Inc., Fort Collins, Colorado, USA) comprising a biodegradable, biocompatible polymer(s) [*i.e*., poly(DL-lactide), poly(DL-lactide-co-glycolide), poly(DL-lactide-co-ε-caprolactone), polycaprolactone, or a combination thereof] in a biodegradable solvent (*i.e*., N-methyl-2-pyrrolidone). The controlled release formulation is generally injected into a patient within 3cm, preferably within 1 cm, and most preferably 0.3 cm, from the skin to control his cancer pain.

It is understood that any homopolymer or copolymer of lactic and glycolic acid can be utilized. The lactic/glycolic acid polymers are solids, wherein the drug and polymers are both dissolved in a biodegradable solvent. After the injection, the biodegradable solvent diffuses out leaving behind the polymer(s) in the form of precipitated, biodegradable particles, which holds most of the drug. As the polymer particles gradually erodes/degrades, the drug is released into the systemic circulation. The release rate of drug is determined by how quickly the polymer particles erodes/degrades in the body.

The active drug may also be incorporated and delivered into the storage site using different methods, such as mixing the drug with the biodegradable, biocompatible polymer(s) in a solvent, evaporating the solvent to obtain polymer particles mixed with the active drug. The size of the drug containing polymer particles should be, small enough to be incorporated (not dissolved) into a suspension in a liquid (preferably an aqueous liquid). The suspension is injected into the patient's tissue proximate the skin surface. The liquid quickly leaves the depot site, leaving behind a polymer implant containing the active drug. The release of active drug from the polymer implant can be increased in the manner described above.

### Example 14

The effects of heating on the release of a drug incorporated in a biocompatible, biodegradable polymer matrix were examined. An anesthetic (*i.e.,* lidocaine) was incorporated into the polymer matrix (*i.e*., lactide/glycolide polymer) to form an anesthetic drug/polymer composition. The anesthetic drug/polymer composition may be used for injecting/planting under the skin of a patient, wherein the drug is gradually released into the body as the polymer matrix slowly erodes in the body.

The anesthetic drug/polymer composition was made by dissolving one tenth of one gram of lactidelglycolide polymer (Medisorb Grade 8515DL, Medisorb Technologies International, L.P., Cincinnati, Ohio, USA) and 0.1 gram of lidocaine base in 2 grams of acetone to form a solution. Approximately 5 mL of water (pH adjusted to above 8) was slowly added into the solution while the solution was stirred by a rapidly rotating Teflon coated magnetic bar. A Medisorb-lidocaine mixture precipitated out as a textured material attached on the magnetic bar and as fine particles suspended in the solution. Approximately 0.5 mL of the solution containing the fine particles were injected into a 0.2 micrometer PTFE filter (Nalgene, 25 mm). Normal saline was infused through the filter via a 3M™ 3000 Modular Infusion Pump at a rate of 2ml/hr for approximately 7 days. This was to wash away the lidocaine that was not incorporated in to the Medisorb matrix and particles smaller than 0.2 micrometer, while lidocaine-polymer particles bigger than 0.2 micrometer were trapped in the filter. The particles slowly degraded due to hydrolysis and thus gradually releases lidocaine to the saline passing through the filter.

A blunt needle was tightly attached to the exit end of the filter, and a thin plastic tube was attached to the blunt needle. Filtered solution from the distal end of the thin plastic tube was collected according the following steps:

| | |
|---|---|
| Step 1: | Filter at room temperature (about 24°C) and collect the filtered solution into a glass vial for approximately 1 hour. |
| Step 2: | Immerse the filter into a 36°C (approximate) water bath, wait approximately 1 hour, and collect the filtered solution from the thin tube for approximately 1 hour. |
| Step 3: | Increase the temperature of the water bath to about 44°C, wait approximately 1 hour, and collect the filtered solution for approximately 1 hour. |
| Step 4: | Take the filter out of the water bath and leave at room temperature (about 24°C) for approximately 0.5 hours, collect the filter solution for approximately 1 hour. |
| Step 5: | Repeat Step 4 after approximately 2 hours. |

Saline was infused through the filter at the 2 mL/hour rate for the entire experiment. The solution coming out of the thin plastic tub during non-collecting time were discarded. Concentrations of lidocaine in above collected solutions were determined by an HPLC (High Performance Liquid Chromatography) method.

Lidocaine release rates from the polymer matrix at different temperatures were calculated from lidocaine concentrations in the collected samples. The release rates are shown in Table E, as follows:

**TABLE E**

| Step | Temperature | Lidocaine Release Rate (mcg/hour) |
|---|---|---|
| 1 | 24°C | 0.36 |
| 2 | 36°C | 0.61 |
| 3 | 44°C | 1.59 |
| 4 | 24°C | 0.47 |
| 5 | 24°C | 0.38 |

As the results demonstrate, the lidocaine release rate increased when temperature at the filter (and hence the temperature of the lidocaine-polymer particles) was increased, and decreased when the temperature was decreased. Although the filter temperature in Steps 4 and 5 were the same, the lidocaine release rate in Step 5 was lower than that in Step 4, and approaches that in Step 1.

Although the total quantities of Medisorb and lidocaine in the filter were not measured, the relative differences in the lidocaine release rates at different temperatures demonstrate that lidocaine release rate from Medisorb polymer increases with temperature. The finding that lidocaine release rate in Step 5 was lower than that in Step 4 suggest that the release rate decreases gradually after the temperature is lowered.

Since the degradation (hydrolysis) of Medisorb polymer is believed to control the release rates, these results suggest that Medisorb polymer degradation rate increases with increasing temperature. This suggests that the release rate of any drug incorporated in the Medisorb matrix (or other similar materials) and injected into the body can be increased by increasing temperature. In addition to increasing hydrolysis rate of the Medisorb-lidocaine particles, heat is also expected to increase the flow of body fluid surrounding the particles in the storage site in actual application, which should cause an additional increase in the drug absorption rate.

Another experiment was conducted on the Medisorb (same type as discussed above). A first sample of the Medisorb (transparent beads) weighing 0.1024 grams was placed in a first glass vial with 9.9024 grams of 0.9% sodium chloride injection solution. The first glass vial was sealed with parafilm and placed in an oven which maintained a temperature of about 43°C. A second sample of the Medisorb weighing 0.1028 grams was placed in a second glass vial with 9.9167 grams of 0.9% sodium chloride injection solution. The second glass vial was sealed with parafilm and placed in a room with a temperature of about 23°C.

After 29 days, few visible change had occurred to the Medisorb held at room temperature (second sample). However, the Medisorb held at about 43°C changed from a transparent material to a milky-white color with smoothed edges. The Medisorb beads also appeared smaller than the original size. This simple experiment demonstrates that the degradation rate of the Medisorb polymer increases with increasing temperature.

### Example 15

Still another example of storage site absorption using the embodiment of the present invention illustrated in FIGs. 1 and 2 consists of a patient or care giver implanting a solid piece (*i.e.*, plate, rod, or the like) made of a biocompatible, bioerodable material(s), such as listed in Example 16, under the skin surface. By way of example, insulin can be incorporated into such a material. The insulin-containing solid piece is implanted into a diabetic patient in a position within 3 cm, preferably within 1 cm, and most preferably within 0.3 cm, from the skin. The insulin release rate from the solid piece is designed to be sufficient to provide the baseline insulin need for extended period of time (*e.g*., a few months). Before each meal, the patient places the temperature control apparatus 100, preferably with a pre-determined heating duration, on to the skin site under which the solid piece resides. The heat from the temperature control apparatus 100 increases the flow of blood or other body fluid surrounding the solid piece, thus increases the erosion/degradation of the solid piece and delivers extra insulin to the systemic circulation to suppress the sugar from the meals. After the pre-determined duration of temperature control apparatus 100 is over or after the patient discontinues the heating from the temperature control apparatus 100, the erosionldegradation rate of the solid piece gradually returns to normal, as does the insulin release rate.

Furthermore, such a system can be used with testosterone in a solid piece which implanted in the patient's skin. Preferably, the temperature control apparatus 100 is designed to last substantially longer (*i.e*., approximately 6-10 hours). The patent applies the temperature control apparatus 100 on the skin site under which the solid piece resides to obtain increased testosterone levels in the blood in the period from morning to evening when testosterone is most needed.

Although only a small number of drugs have been disclosed in Examples 13-18, any drug used in a treatment that fits the following description may potentially benefit from the methods: 1) the treatment requires that the drug have a baseline deliver rate over long treatment duration (such as longer than a day, preferably over a week), and 2) the treatment requires the drug to have increased delivery rates for a period or periods of time during the long treatment duration. A variety of drugs and drug classes can be utilized with such treatments. The drugs include, but are not limited to, nicotine, testosterone, estradiol, nitroglycerin, clonidine, dexamethasone, tetracaine, lidocaine, fentanyl, sufentanil, progestrone, insulin, prilocaine, bupivacaine, sumatriptan, and dihydroergotamine. The drug classes include, but are not limited to, androgen, estrogen, non-steroidal anti-inflammatory agents, anti-hypertensive agents, analgesic agents, anti-depressants, antibiotics, anti-cancer agents, local anesthetics, antiemetics, anti-infectants, contraceptives, anti-diabetic agents, steroids, anti-allergy agents, anti-migraine agents, agents for smoking cessation, anti-asthma agents, and anti-obesity agents.

### Example 16

Still yet another example of storage site absorption using the embodiment of the present invention illustrated in FIGs. 1 and 2 consists of a patient or care giver imbedding a drug into the depot site. By way of example, a care giver can embed an anti-migraine drug, such as a powder form of dihydroergotamine, sumatriptan, or ergotamine, by hitting the drug into a depot site under the skin at high speed (such as by a device manufactured by Powderject Pharmaceutical, United Kingdom) when a patient feels an episode of migraine headache is imminent. With the Powderjet device, the drug powder is accelerated to a speed higher than the speed of sound and hit into the skin. A temperature control apparatus 100, preferably lasting approximately 1 hour, is immediately applied on the skin over the location of the embedded drug. The heat from the temperature control apparatus 100 increases the speed of the body fluid flow surrounding the anti-migraine drug and carries the anti-migraine drug into the systemic circulation faster. As a result, therapeutical blood concentrations of the anti-migraine drug is reached earlier and in time to treat the migraine headache.

This technique may also be used to deliver a preventative baseline release rate of a drug, such as anti-migraine drug or nitroglycerine. A heating patch is then applied to release extra drug when a medical episode begins.

It is, of course, understood that the heating devices discussed above could be replaced by an infrared heating device or a microwave heating device with a feedback mechanism. All the controls and variations in controls discussed above would apply to such devices.

### Example 17

Ultrasound can be used to increase release rate of injected controlled release drug formulations, particularly, when the controlled release formulations are in the form of relatively large particles (*i.e*., 25 µm or larger). The controlled release formulation is injected into the patient's tissues within 3 cm, preferably within 1 cm, and most preferably 0.3 cm from the skin. The erosion/degradation rate of the particles determines the rate of release of the drug, and the steady state release rate of the drug is designed to deliver a therapeutical level of drug to the patient. For analgesic drugs, the steady state release rate is usually slightly below that needed to treat an average person's post-operative pain. For a particular patient in whom the steady state release rate is not sufficient (because of his pharmacokinetics and/or level of pain), an ultrasound is directed into formulation and breaks the particles into smaller ones (this requires that the particles are capable of being broken by ultrasound).

This increases the surface area of the formulation exposed to the surrounding body fluid, and hence increases the release rate for the rest of the administration. This method allows the administration of a low release rate formulation which is safe, and then increasing the release rate for patients who need higher delivery rates. The intensity, frequencies, and duration of ultrasound can be chosen to increase the release rate to proper levels. Exemplary ultrasound treatment and devices can be found in U.S. Patent 4,948,587 issued August 14, 1998 to Kost et al., hereby incorporate herein by reference.

### Example 18

The generation of an electric potential on a portion of a patient's body can be used to increase release rate of injected controlled release drug formulations, particularly, when the controlled release formulations exist in ionized form in the formulations and/or surrounding body fluid. For example, when a controlled release insulin is injected into a diabetic patient's skin, the normal release rate of insulin from this formulation is controlled by the dissolution rate of the particles in which insulin resides wherein the normal release rate provides an adequate baseline insulin level in the patient. As shown in FIG. 26, the patient places a first electrode 262 on the skin 134 over the injection site of the controlled release insulin formulation 264. A second electrode 266 is placed on a skin 134 in a position near the injection site of the controlled release insulin formulation 264 (i.e., at least a few centimeters away). Before each meal when the patient needs to increase his blood insulin level to suppress sugar from the meal, the patient connects the first electrode 262 and the second electrode 266 with wires 268 and 270, respectively, to an electric current generating device 272. The electric current generating device 272 introduces an electrical potential between the first electrode 262 and the second electrode 266. Preferably, with the use of insulin, the electrical amperage should be in the range of between about 0.2 and 4 mA. Because at the physiological pH, insulin molecules carry net negative electric charges, the first electrode 262 should have a negative charge which pushes the negatively charged insulin away from the body fluid surrounding the formulation and into the systemic circulation 254. This makes the insulin release faster. Preferably, the intensity and duration of the current can be altered with the electric current generating device 272 to deliver the requisite therapeutic amount of extra insulin.

### Example 19

The generation of a vibration over the injection site of controlled release drug formulations can be used to increase release rate of the formulations, particularly, when the controlled release formulations have limited solubility in body fluid or with solid formulations whose erosion/degradation speed can be significantly increased by increasing flow/exchange of body fluid surrounding the solid formulation. For example, when a controlled release insulin is injected into a diabetic patient's skin, the normal release rate of insulin from this formulation is controlled by the erosion/degradation or dissolution rate of the particles in which insulin resides wherein the normal release rate provides an adequate baseline insulin level in the patient. As shown in FIG. 27, before each meal, the patient places a vibration generating device 282 on the skin 134 over the injection site of the controlled release insulin formulation 264. The vibration generating device 282, preferably, delivers vibration of between about 20 and 400 Hz. The vibration agitates the body fluid (not shown) surrounding the controlled release insulin 264 and increases its circulation. As a result, more insulin is released from the controlled release insulin formulation 264 to the systemic circulation 254 shortly before the meal to suppress the sugar from the meal. Preferably, the intensity and duration of the vibration can be altered with the vibration generating device 282 to deliver the requisite therapeutic amount of extra insulin.

Although only a few drugs have been disclosed in Examples 19-22, any drug used in a treatment that fits the following description may potentially benefit from the physical methods for inducing increased release: 1) the treatment requires that the drug have a baseline deliver rate over long treatment duration (such as longer than a day, preferably over a week), 2) the treatment requires the drug to have increased delivery rates for a period or periods of time during the long treatment duration, and 3) the formulations respond to the one or more of the physical methods for inducing increased release. A variety of drugs and drug classes can be utilized with such treatments. The drugs include, but are not limited to, nicotine, testosterone, estradiol, nitroglycerin, clonidine, dexamethasone, tetracaine, lidocaine, fentanyl, sufentanil, progestrone, insulin, prilocaine, bupivacaine, sumatriptan, and dihydroergotamine. The drug classes include, but are not limited to, androgen, estrogen, non-steroidal anti-inflammatory agents, anti-hypertensive agents, analgesic agents, anti-depressants, antibiotics, anti-cancer agents, local anesthetics, antiemetics, anti-infectants, contraceptives, anti-diabetic agents, steroids, anti-allergy agents, anti-migraine agents, and agents for smoking cessation.

### Example 20

Another example of the present invention comprises using a temperature control apparatus 300, similar to that shown in FIG. 23, which is capable of heating and cooling, such that the rate of absorption of injected controlled release drug formulation can be increased or decreased, as needed.

For example, when a controlled release drug formulation is injected into a patient's skin, the normal release rate of the drug from this formulation is controlled by the erosion/degradation rate of the particles in which the drug resides wherein the normal release rate provides an adequate baseline drug level in the patient. As shown in FIG. 28, if the level of the drug in the patient's system requires adjusting, the temperature control apparatus 300 is placed on the skin 134 over the injection site of the controlled release drug formulation 302. Heating will result in an increase in drug absorption (as previously discussed) and cooling will reduce drug absorption to prevent overdose. FIG. 23 illustrates the temperature control apparatus 300 as a thermoelectric module which is be used for both heating or cooling. The temperature control apparatus 300 functions as a small heat pump, wherein a low voltage DC power source 304 provides a current in one direction 306 to a thermoelectric unit 310 which results in heating on a first side 308 (preferably a ceramic substrace) of the temperature control apparatus 300 and cooling on a second side 312 (preferably a finned dissipation structure) of the temperature control apparatus 300. If the current direction is reversed, the first side 308 will cool and the second side will heat. The temperature control apparatus 300 may be control with a closed loop temperature controller, as shown previously in FIG. 24.

A variety of drugs and drug classes can be utilized with such treatments. The drugs include, but are not limited to, nicotine, nitroglycerin, clonidine, dexamethasone, fentanyl, sufentanil, and insulin. The drug classes include, but are not limited to, androgen, non-steroidal anti-inflammatory agents, anti-hypertensive agents, analgesic agents, anti-depressants, anti-cancer agents, anti-diabetic agents, steroids, anti-migraine agents, and agents for smoking cessation.

### Example 21

Another example of the present invention comprises using the temperature control apparatus 300, as shown in FIG. 23, or any device which is capable of cooling the skin in conjunction with an injectable liquid drug delivery formulation containing thermal gel.

The main difference between a thermal gel and a regular gel is that a thermal gel is a liquid in room temperature (*i.e*., about 20-25°C) and is a gel at body temperature (*i.e*., about 37°C), whereas, with regular gel, the viscosity of the gel generally lowers with increasing temperature. Thus, while the thermal gel is at room temperature (*i.e.,* in liquid form), a drug formulation is mixed into the thermal gel. The thermal gel/drug mixture may then be easily drawn into a syringe and injected to the patient. Once in the patient's body, the thermal gel/drug mixture quickly solidifies into a gel. The gel then dissolves over time releasing the drug formulation into the patient systemic circulation.

Using a cooling device, such as the temperature control apparatus shown in FIG. 23, the thermal gel/drug mixture which has solidified under the skin can be cooled to revert the gel back into a liquid. In a liquid state, the drug formulation diffusion rate and release rate increase, thereby increasing the drug formulation present in the patient's systemic circulation when needed.

An example of a thermal gel is Smart Hydrogel™ developed by Gel SciencelGelMed and consists of an entangled network of two randomly grafted polymers. One polymer is poly(acrylic acid) which is bioadhesive and pH-responsive. The other polymer is a triblock copolymer containing poly(propylene oxide) ("PPO") and poly(ethylene oxide) ("PEO") segments in the sequence PEO-PPO-PEO.

An example of using the present invention with a thermal gel is the delivery of additional insulin to a diabetic patient prior to the intake of food. The thermal gel containing the insulin can be injected subcutaneously in order to form a gel to release a continuous baseline dosage of insulin. At a meal when insulin is needed to absorb extra sugar in the circulation, the patient can apply the cooling device on the skin adjacent the injection site and cool the injection site to a temperature below the gelling temperature of the thermal gel/insulin mixture. The gel will, of course, become a liquid and increase the insulin level in the patient's body to compensated for the ingested meal. This process can be repeated many times until the injected thermal gel/insulin mixture is gone. The advantage of this drug delivery system is that the diabetic patient can control insulin delivery during the course of a few days, even a few weeks, with only one injection.

### Example 22

As shown in FIG. 29, an insulating material can be incorporated with the controlled temperature apparatus to assist in not only minimizing the temperature variation, but also increasing the temperature of the DDDS and the skin under it (by decreasing heat loss), each of which tend to increase dermal drug absorption.

FIG. 29 illustrates a configuration similar to that illustrated in FIG. 4 wherein the temperature control apparatus 100 of FIG. 2 is attached to the DDDS 120 of FIG. 3. The DDDS 120 attached to a portion of the skin 134 of a patient. An insulating sleeve 350 abuts the skin 134 and encases a substantial portion of the temperature control apparatus 100 and the DDDS 120.

FIG. 30 illustrates another insulating sleeve 360 made of an insulating material, such as closed-cell foam tape, with adhesive edges 362 attached to a patient's skin 134, slightly larger than and covering a DDDS 364. FIG. 31 illustrates the insulating, sleeve 360 covering a heating apparatus 366 and the DDDS 364 attached to a patient's skin 134. FIG. 32 illustrates the insulating sleeve 360 covering an area over the skin 134 where an injected/implanted/controlled/extended release drug formulation 368 has been located.

### Example 23

Another application of the present invention involves the use of a heating device, such as discussed above, in conjunction with a typical liquid drug injection. For some drugs, increased speed of absorption into the systemic circulation after they are injected into the body may provide treatment to the patients. For instance, to be effective, the anti-migraine drug, dihydroergotamine, must reach an effective concentration level in the blood stream within a certain amount of time from the onset of the migraine attack or the drug will be ineffective. Currently, a drug's absorption into the patient's systemic circulation cannot be altered after it is injected. Thus, the controlled heating aspect of the present invention can be used to increase the absorption speed of subcutaneously and intramuscularly injected drugs.

For example, after a drug is injected subcutaneously or intramuscularly, a heating patch, such as described in the above examples, may be placed on the skin under which the injected drug resides. The heating increases the circulation of body fluid surrounding the injected drug, increases the permeability of blood vessel walls in the surrounding tissue, and, thus, results in increased speed of absorption of the drug into the systemic circulation.

Such a method would be useful for drugs which are injected into a part of the body that can be heated by a heating means on or outside the skin and whose effect can be improved by increased absorption speed into the systemic circulation or deeper tissues. Such drugs may include; anti-migraine agents, anti-hypertensive agents, analgesics, antiemetics, cardiovascular agents. Specific drugs may include dihydroergotamine, ergotamine, sumatriptan, rizatriptan, zolmitriptan, and other selective 5-hydroxytryptamine receptor subtype agonists, morphine and other narcotic agents, atropine, nitroglycerin, fentanyl, sufentanil, alfentanil, and meperidine.

Since increased absorption speed into the systemic circulation usually can cause higher peak concentrations in the blood, this technology may also be used to increase peak blood concentrations of drugs that are injected subcutaneously and intramuscularly.

Some drugs need to be injected intravenously because systemic absorption for subcutaneous and intramuscular injections take too long to take effect. However, intravenous injection is more difficult to perform and involves more risks. With the use of the present invention, the absorption speed of some drugs may be increased enough so that subcutaneous or intramuscular injection can provide sufficient speed of absorption. Therefore, this technology may also be used for replacing intravenous injections with subcutaneous or intramuscular injections for some drugs.

As a specific example, a patient may inject himself with sumatriptan or dihydroergotamine subcutaneously after he feels a migraine attack. He then removes a heating patch containing a heat generating medium comprising iron powder, activated carbon, water, sodium chloride, and sawdust (similar to Example 1) out of its air-tight container and places it over the injection site. The heating patch quickly increases the temperature of the skin under the heating patch into a narrow range of 39-43°C and maintains it there for at least 15 minutes. The circulation speed of the body fluid surrounding the injected drug and the permeability of the blood vessels in the surrounding tissues are both increased by the heating. As a result, the drug enters the systemic circulation and reaches the acting site more rapidly, and the patient receives more rapid and/or better control of the migraine attack.

In another example, a nurse can inject morphine into a patient's muscle tissue to treat severe pain. The nurse then places a heating patch, as describe above, over the injection site. The speed of morphine absorption into the systemic circulation is increased as previously discussed. As a result, the patient receives more rapid and/or better pan control.

### Example 24

Another application of the present invention involves the use of a heating device, such as discussed above, to mimic circadian patterns. For example, testosterone or its derivatives, such as testosterone enanthate and testosterone cypionate, can be injected intramuscularly into men to substitute or replace diminished or absent natural testicular hormone. Testosterone enanthate and testosterone cypionate are preferred over testosterone, as they have longer duration of action than testosterone. However, it is understood that testosterone or its derivative, such a testosterone ester, may be incorporated into a controlled release polymer matrix, such as homopolymer or copolymer of lactic and glycolic acid, preferably poly(DL-lactide), poly(DL-lactide-co-glycolide), and poly(DL-lactide-co-(-caprolactone)), to increase the duration of action. Following intramuscular injection, testosterone enanthate is absorbed gradually from the lipid tissue phase at the injection site to provide a duration of action of up to 2-4 weeks. However, natural blood testosterone concentrations in healthy man are higher in a day and lower in the night. So blood testosterone concentrations obtained from injected testosterone derivatives do not mimicking the natural circadian pattern.

By way of example, a patient can inject testosterone enanthate either subcutaneously or intramuscularly (if intramuscularly, the injection should be relatively close to the skin surface). The patient then places a heating patch on the injection site every morning (until all the injected testosterone enanthate is depleted). The heating patch quickly increases the temperature of the injection site to a narrow range, and maintains it therefore a desirable duration of time (i.e., about 8 hours). They heating causes increased release of testosterone enanthate and/or increased rate of conversion from testosterone enanthate to testosterone, and, thus, higher blood testosterone concentrations. The "used-up" patch is removed before a new heating patch is placed on the same. Using this intermittent heat application technique, blood testosterone concentrations are low in the night and high in the day, thus mimicking the natural circadian pattern.

The Androderm® patch manufactured by TheraTech, Inc. is an androgen transdermal therapeutic system similar to the ATTS in Figure 3 and Figure 13. The Androderm® patch is capable of providing testosterone transdermally to a patient for a 24 hour period. The Androderm® system provides a drug formulation reservoir defined by a polyester ethylene vinyl acetate laminate film backing secured along its perimeter to the perimeter of a microporous polyethylene film. The two films are secured to each other in such a way as to form a drug formulation reservoir between the two films. A drug formulation containing testosterone resides in the reservoir. The microporous polyethylene film is permeable and allows the drug formulation to migrate across the microporous membrane and be absorbed into skin, when the drug reservoir is attached to the skin of the patient. When in storage, a drug formulation is prevented from crossing the microporous polyethylene film by a sealed disc of ethylene vinyl acetate copolymer film. The disc acts as a physical barrier to the migration of the drug formulation. The disc is attached to a polyester film release liner. The release liner preserves adhesive disposed on the bottom of the microporous film and the disc prevents the drug formulation from crossing the membrane while the Androderm® patch is in storage. The release liner and disc are removed prior to administration of the patch.

Skin irritation is a major problem with ATTSs currently on the market. The skin irritation is believed to be mainly caused by a permeation enhancer in the formulation. (In the case of Androderm®, the permeation enhancer is monoglyceral oleate). The permeation enhancer is needed to increase skin permeability so that sufficient testosterone can permeate across the skin. The degree of skin irritation is usually positively correlated with the permeation enhancer concentration in the formulation and contact time with the skin. Since controlled heat can significantly increase dermal skin absorption, it is conceivable that, with controlled heat, one may be able to reduce the concentration of the enhancer in the formulation, reduce the contact time between the formulation and the skin, and/or use a milder permeation enhancer. Properly doing so should reduce skin irritation while still delivering sufficient testosterone. In other words, using controlled heat can shift at least some burden to enhance skin permeability from permeation enhancer to heat, which is a much safer way to enhance skin permeability.

The release rate from such testosterone patches (ATTS's) is usually substantially constant during day and night. This is not the natural circadian pattern. Controlled heat can be used to obtain a natural circadian pattern while using an ATTS having a more or less constant release rate. In the morning the user places a heating patch on the skin area under which the injected/implanted controlled release formulation resides. The increased temperature increases the body fluid flow surrounding the formulation and/or increases the erosion speed of the formulation matrix, resulting in increased androgen release rates and thus higher serum levels. The heating is designed to last long enough to maintain higher release rates during the day. In the afternoon or evening, the heating patch stops generating heat (or is removed), and release rates return to unheated levels. The user repeats this everyday. In this arrangement, a blood testosterone concentration profile that mimics the natural circadian pattern can be obtained from otherwise constant release formulations.

### Example 25

Another example of using the embodiment of the present invention illustrated in FIGs. 8-12 for dermally administering testosterone to increase and optimize the amount of drug delivered consists of a user placing ATTS 160, 165, such as a once a day dermal testosterone patch, for example Androderm® produced by TheraTech, Inc. of Salt Lake City, Utah, USA, on the skin 134. The ATTS 160, 165 is generally applied to the skin 134 at night, for example at 10 PM. The user puts the temperature control apparatus 150 on top of the ATTS 160, 165 first thing next morning. The increased temperature in the ATTS 160, 165, the skin 134 and tissues under the skin significantly increase the dermal absorption of testosterone to achieve increased delivery rates for the drug time. In addition, the ATTS 160, 165 a has permeation enhancer, such as glycerol monooleate. In a testosterone ATTS 160, a permeation enhancer is usually necessary for delivering sufficient testosterone, however, permeation enhancers such as glycerol monooleate used in Androderm® may cause serious skin irritation. With potentially less risk of irritation, the increased absorption of testosterone offered by the controlled heating may allow the reduction of the concentration of permeation enhancer which is used in the ATTS 160, 165. The heat should also make the enhancer permeate the skin faster, thus making it more effective. The ultimate result of controlled heat is that the user gets sufficient testosterone from ATTS 160, 165 when it is most needed, during the day time.

### Example 26

Still another example of using the embodiment of the present invention illustrated in FIGs. 8-12 comprises using the temperature control apparatus 150 for increasing absorption rate and decreasing onset time of an androgen material from the ATTS 160, 165. By way of example with the use of a commercially available testosterone patch, such as Androderm-50®, as the ATTS 160, 165, the patient or care giver places the ATTS 160, 165 on the skin 134 of the patient and places the temperature control apparatus 150 over the ATTS 160. Preferably, the temperature control apparatus 150 includes a sufficient amount of heat generating medium for temperature regulating mechanism 108 to sustain an exothermic reaction for at least four hours.

The heat from the temperature control apparatus 150 increases the temperature at a contact surface of the skin 134 and the ATTS 160, 165 to a narrow temperature range between about 38°C and 45° C, preferably between about 39°C and 43° C, more preferably about 42° C and maintains this temperature for a period of time (i.e., approximately four hours). During this time, the heat increases the speed of testosterone release from the ATTS 160, 165, the permeation rate across the skin 134, and the speed of blood circulation which carries testosterone into the systemic circulation faster. After the exothermic reaction ceases (approximately four hours), the testosterone absorption and concentration in the bloodstream begins to decrease from the elevated levels caused by the heat from the ATTS 160, 165 returns to normal (unheated) levels. The patient continues to wear the system for the next 24 hours. Compared with ATTS 160, 165 without the use of the temperature control apparatus 150, the testosterone begins to appear in the bloodstream significantly earlier to yield a shortened onset time and the testosterone concentrations in the bloodstream in the early hours of application are significantly higher than that produced by an unheated ATTS 160, 165. As a result, high serum testosterone levels are achieved in earlier hours (i.e. 4-6 hours after application first thing in the morning).

Having thus described in detail preferred embodiments of the present invention, it is to be understood that the invention defined by the appended claims is not to be limited by particular details set forth in the above description as many apparent variations thereof are possible without departing from the scope thereof.

## Claims

1. A dermal drug delivery system comprising:
a temperature control component (20) capable of generating heat controlled by exposing an oxygen activated exothermic medium (34) within the component;
a drug delivery component (4), the drug delivery component integrated with the temperature control component (20), wherein the drug delivery component (4) comprises a substantially two dimensional drug formulation layer (10) comprising at least one pharmaceutically active substance;
a barrier film (16) substantially impermeable to oxygen and alcohol, the film (16) having dimensions slightly larger than said drug formulation layer (10) and having an upper and lower side;
a means for preventing transfer of substances between said temperature control component (20) and said drug delivery component (4), said means including a tray (8) and the barrier film (16), said tray (8) being substantially impermeable to alcohol and water and having a flat rim (44) and a shallow reservoir (40) and being configured for removal prior to application of the system to a skin surface;
a means (18) to affix the integrated drug delivery component onto a patient's skin such that the skin is in contact with said formulation layer; and
a means (6) to shield the integrated temperature control component (20) and drug delivery component (4) from oxygen and moisture,
wherein the drug formulation layer (10) is disposed within the shallow reservoir (40) of the tray and the barrier film (16) covering the drug formulation layer (10) and the barrier film (16) is sealed to the rim (44) of the tray (8) such that the drug formulation layer (10) is substantially isolated within the reservoir (40) of the tray (8), the two dimensional reservoir with an exothermic medium (34) being secured to the upper side of the barrier film (16), and the drug formulation layer (10) being affixed to the lower side of the barrier film (16).

2. The system as claimed in claim 1, wherein said oxygen activated exothermic medium (34) comprises activated carbon, iron and water.

3. The system as claimed in claim 1, wherein the temperature control component (20) includes a cover (28) having selected air permeability comprising a layer of material substantially impermeable to air, said layer defining a selected number of holes (32) of selected sizes.

4. The system as claimed in claim 1, said drug delivery component (4) comprising a drug formulation layer (10) disposed between a backing film and a rate-limiting membrane (130).

5. The system as claimed in claim 1, said drug formulation layer (10) disposed between a backing film and non rate-limiting membrane (130).

6. The system as claimed in claim 1, wherein said temperature control component (20) and said drug delivery component (4) are isolated from one another such that vapors are not transferred between the components within a common packaging.

7. The system as claimed in claim 1, said drug delivery component (4) comprising a drug selected from the group of fentanyl, sufentanil, carfentanil, testosterone, lidocaine, tetracaine, prilocaine, lopivacaine, bupivacaine, procaine, flurbiprofen, naproxen, ibuprofen, scopolamine, nicotine and dexamethasone.

8. The system as claimed in claim 1, said drug delivery component (4) comprising a drug selected from the classes of analgesics, anti-inflammatory agents, steroids, androgens, estrogens, hormones, anti-viral agents, anti-asthma agents, cardiovascular agents, anti-hypertension agents, antidepressants, and cox-2 inhibitors.

9. The system of claim 3, wherein the selected number of holes (32) are removably covered with rate limiting hole covers (54).

10. The system of claim 1, wherein the barrier film sealed to the rim is manually separable.

11. A method for preparing a dermal drug delivery system employing controlled heat as specified in claim 1, the method comprising the steps of:
disposing a drug formulation layer (10) within a formulation reservoir (40) of a tray (8) having a flat rim (44), the tray (8) being substantially impermeable to alcohol and water;
securing the drug formulation layer (10) within the tray (8) to a drug applicator;
securing a temperature control component (20) to the drug applicator; and
isolating the drug formulation layer (10) within the tray (8) with a barrier film (16) that covers the drug formulation layer (10) and is sealed to the rim (44) of the tray (8) such that the temperature control component (20) and the drug formulation layer (10) are isolated from each other, the temperature control component (20) secured to an upper side of the barrier film (16) and the drug formulation layer (10) secured to a lower side of the barrier film.

12. A method of claim 11, wherein the step of securing the drug formulation layer (10) comprises:
impregnating a layer of fabric material with a boric acid, sodium salt; and
placing said fabric material in contact with said formulation, said formulation containing polyvinyl alcohol, to cause the drug formulation to solidify as a gel onto the fabric material.

13. The method of claim 12, wherein the step of isolating the drug formulation layer (10) comprises:
placing a heat sealable barrier film (16) on top of the fabric material and sealing said barrier film (16) to the tray (8) and the fabric material.

14. The method of claim 13, wherein a portion of the barrier film (16) is not sealed onto the tray (8) in order to provide a leading edge for peeling.

15. The method as claimed in claim 13, said drug formulation layer (10) comprising a drug selected from the group of fentanyl, sufentanil, carfentanil, testosterone, lidocaine, tetracaine, prilocaine, lopivacaine, bupivacaine, procaine, flurbiprofen, naproxen, ibuprofen, scopolamine, nicotine and dexamethasone.

16. The method as claimed in claim 13, said drug formulation layer (10) comprising a drug selected from the classes of analgesics, anti-inflammatory agents, steroids, androgens, estrogens, hormones, anti-viral agents, anti-asthma agents, cardiovascular agents, anti-hypertension agents, antidepressants, and cox-2 inhibitors.

## Patentansprüche

1. System zur Verabreichung von Medikamenten für die Haut, umfassend:
eine Temperatureinstellkomponente (20), die in der Lage ist, Wärme zu erzeugen, welche durch Freisetzen eines sauerstoffaktivierbaren exothermen Mediums (34) innerhalb der Komponente eingestellt wird;
eine Medikamentenzuführkomponente (4), wobei diese in der Temperatureinstellkomponente (20) integriert ist, wobei die Medikamentenzuführkomponente (4) eine im Wesentlichen zweidimensionale Medikamentenformulierungsschicht (10) umfasst, welche wenigstens eine pharmazeutisch aktive Substanz umfasst; einen Barrierefilm (16), der für Sauerstoff und Alkohol im Wesentlichen undurchlässig ist, wobei der Film (16) Abmessungen aufweist, die etwas größer sind als die der Medikamentenformulierungsschicht (10), und eine Oberseite und eine Unterseite aufweist;
ein Mittel zur Verhinderung der Übertragung von Substanzen zwischen der Temperatureinstellkomponente (20) und der Medikamentenzuführkomponente (4), wobei das Mittel einen Behälter (8) und den Barrierefilm (16) enthält, wobei der Behälter (8) im Wesentlichen für Alkohol und Wasser undurchlässig ist und einen ersten Flachring (44) und ein flaches Reservoir (40) aufweist und zur Entfernung vor der Anwendung des Systems auf einer Hautoberfläche ausgelegt ist;
ein Mittel (18), um die integrierte Medikamentenzuführkomponente (4) an der Haut eines Patienten so anzubringen, dass die Haut sich im Kontakt mit der Formulierungsschicht befindet; und
ein Mittel (6) zur Abschirmung der integrierten Temperatureinstellkomponente (20) und der Medikamentenzuführkomponente (4) gegenüber Sauerstoff und Feuchtigkeit,
wobei die Medikamentenformulierungsschicht (10) innerhalb des flachen Reservoirs (40) des Behälters angeordnet ist und der Berrierefilm (16) die Medikamentenformulierungsschicht (10) bedeckt und der Barrierefilm (16) mit dem Ring (44) des Behälters (8) so verbunden ist, dass die
Medikamentenformulierungsschicht (10) im Wesentlichen innerhalb des Reservoirs (40) des Behälters (8) isoliert ist, wobei das zweidimensionale Reservoir mit einem exothermen Medium (34) an der Oberseite des Barrierefilms (16) befestigt ist, und wobei die Medikamentenformulierungsschicht (10) an der Unterseite des Barrierefilms (16) angebracht ist.

2. System nach Anspruch 1,
bei dem das Sauerstoff aktivierbare exotherme Medium (34) Aktivkohle, Eisen und Wasser umfasst.

3. System nach Anspruch 1,
bei dem die Temperatureinstellkomponente (20) eine Abdeckung (28) mit ausgewählter Luftdurchlässigkeit aufweist, welche eine Schicht aus Material umfasst, welches für Luft im Wesentlichen undurchlässig ist, wobei die Schicht eine ausgewählte Anzahl Löcher (32) mit ausgewählten Größen definiert.

4. System nach Anspruch 1,
bei dem die Medikamentenzuführkomponente (4) eine Medikamentenformulierungsschicht (10) aufweist, welche zwischen einem Stützfilm und einer durchsatzbegrenzenden Membran (130) angeordnet ist.

5. System nach Anspruch 1,
bei dem die Medikamentenformulierungsschicht (10) zwischen einem Stützfilm und einer nicht durchsatz-begrenzenden Membran (130) angeordnet ist.

6. System nach Anspruch 1,
bei dem die Temperatureinstellkomponente (20) und die Medikamentenzuführkomponente (4) derart voneinander isoliert sind, dass Dämpfe zwischen den Komponenten innerhalb einer gemeinsamen Verpackung nicht übertragen werden können.

7. System nach Anspruch 1,
wobei die Medikamentenzuführkomponente (4) ein Medikament umfasst, welches aus folgender Gruppe ausgewählt ist: Fentanyl, Sufentanil, Carfentanil, Testosteron, Lidocain, Tatracain, Prilocain, Lopivacain, Bupivacain, Procain, Flurbiprofen, Naproxen, Ibuprofen, Scopolamin, Nikotin und Dexamethason.

8. System nach Anspruch 1,
wobei die Medikamentenzuführkomponente (4) ein Medikament umfasst, welches aus den folgenden Klassen ausgewählt ist: Analgetika, Anti-Entzündungs-Mittel, Steroide, Androgene, Östrogene, Hormone, Anti-Viren-Mittel, Anti-Asthma-Mittel, Herzkreislaufmittel, Blutdruck senkende Mittel, Antidepressiva und Cox-2-Hemmer.

9. System nach Anspruch 3,
bei dem die ausgewählte Anzahl Löcher (32) mit entfernbaren durchsatzbegrenzenden Lochabdeckungen (54) abgedeckt sind.

10. System nach Anspruch 1,
bei welchem der am Ring befestigte Barrierefilm manuell abtrennbar ist.

11. Verfahren zum Bereitstellen eines Systems nach Anspruch 1, zur Verabreichung von Medikamenten für die Haut unter kontrolliertem Wärmeeinsatz, welches folgende Schritte umfasst:
Anordnen einer Medikamentenformulierungsschicht (10) innerhalb eines Formulierungsreservoirs (40) eines Behälters (8), der einen flachen Ring (44) aufweist, wobei der Behälter (8) im Wesentlichen undurchlässig für Alkohol und Wasser ist;
Befestigen der Medikamentenformulierungsschicht (10) innerhalb des Behälters (8) an einer Medikamentenabgabeeinrichtung;
Befestigen einer Temperatureinstellkomponente (20) an der Medikamentenabgabeeinrichtung; und
Isolieren der Medikamentenformulierungsschicht (10) innerhalb des Behälters (8) mit einem Barrierefilm (16), welcher die Medikamentenformulierungsschicht (10) abdeckt und mit dem Ring (44) des Behälters (8) so verbunden wird, dass die Temperatureinstellkomponente (20) und die Medikamentenformulierungsschicht (10) voneinander isoliert werden, wobei die Temperatureinsstellkomponente (20) an der Oberseite des Barrierefilms (16) befestigt wird und die Medikamentenformulierungsschicht (10) an der Unterseite des Barrierefilms befestigt wird.

12. Verfahren nach Anspruch 11,
bei dem der Schritt des Befestigens der Medikamentenformulierungsschicht (10) folgendes umfasst:
Imprägnieren einer Schicht aus Gewebematerial mit Borsäure, Natriumsalz; und
in Kontakt Bringen des Gewebematerials mit der Formulierung, wobei die Formulierung Polyvinylalkohl enthält, damit die Medikamentenformulierung sich auf dem Gewebematerial zu einem Gel verfestigt.

13. Verfahren nach Anspruch 12,
bei dem der Schritt des Isolierens der Medikamentenformulierungsschicht (10) umfasst:
Anordnen eines heißsiegelfähigen Barrierefilms (16) auf dem Gewebematerial und Verbinden des Barrierefilms (16) mit dem Behälter (8) und mit dem Gewebematerial.

14. Verfahren nach Anspruch 13,
bei dem ein Teil des Barrierefilms (16) nicht mit dem Behälter (8) verbunden ist, um eine Ablösekante vorzusehen.

15. Verfahren nach Anspruch 13,
bei dem die Medikamentenformulierungsschicht (10) ein Medikament umfasst, welches aus folgender Gruppe ausgewählt ist: Fentanyl, Sufentanil, Carfentanil, Testosteron, Lidocain, Tatracain, Prilocain, Lopivacain, Bupi-vacain, Procain, Flurbiprofen, Naproxen, Ibuprofen, Scopolamin, Nikotin und Dexamethason.

16. Verfahren nach Anspruch 13,
bei dem die Medikamentenformulierungsschicht (10) ein Medikament umfasst, welches aus den folgenden Klassen ausgewählt ist: Analgetika, Anti-Entzündungs-Mittel, Steroide, Androgene, Östrogene, Hormone, Anti-Viren-Mittel, Anti-Asthma-Mittel, Herzkreislaufmittel, Blutdruck senkende Mittel, Antidepressiva und Cox-2-Hemmer.

## Revendications

1. Système de délivrance de médicament dermique comprenant:
un composant de régulation de la température (20) capable de générer de la chaleur régulée by exposition d'un milieu exothermique activé par l'oxygène (34) dans le composant;
un composant de délivrance de médicament (4), le composant de délivrance de médicament intégré avec le composant de régulation de la température (20), où le composant de délivrance de médicament (4) comprend une couche de formulation de médicament (10) sensiblement bidimensionnelle comprenant au moins une substance pharmaceutiquement active;
un film formant barrière (16) sensiblement imperméable à l'oxygène et l'alcool, le film (16) ayant des dimensions légèrement plus grandes que ladite couche de formulation de médicament (10) et ayant un côté supérieur et un côté inférieur ;
un moyen pour empêcher le transfert de substances entre ledit composant de régulation de la température (20) et ledit composant de délivrance de médicament (4), ledit moyen incluant un plateau (8) et le film formant barrière (16), ledit plateau (8) étant sensiblement imperméable à l'alcool et l'eau et ayant un bord plat (44) et un réservoir peu profond (40) et étant configuré pour le retrait avant l'application du système à une surface de la peau ;
un moyen (18) pour fixer le composant de délivrance de médicament intégré sur la peau d'un patient de sorte que la peau est en contact avec ladite couche de formulation; et
un moyen (6) pour protéger le composant de régulation de la température (20) et le composant de délivrance de médicament (4) intégrés contre l'oxygène et l'humidité,
où la couche de formulation de médicament (10) est disposée dans le réservoir peu profond (40) du plateau et le film formant barrière (16) couvrant la couche de formulation de médicament (10) et le film formant barrière (16) est soudé au bord (44) du plateau (8) de sorte que la couche de formulation de médicament (10) est sensiblement isolée dans le réservoir (40) du plateau (8), le réservoir bidimensionnel avec un milieu exothermique (34) étant fixé au côté supérieur du film formant barrière (16), et la couche de formulation de médicament (10) étant fixée au côté inférieur du film formant barrière (16).

2. Système selon la revendication 1, où ledit milieu exothermique activé par l'oxygène (34) comprend du carbone activé, du fer et de l'eau.

3. Système selon la revendication 1, où le composant de régulation de la température (20) inclut un couvercle (28) ayant une perméabilité à l'air choisie comprenant une couche de matière sensiblement imperméable à air, ladite couche définissant un nombre choisi de trous (32) de tailles choisies.

4. Système selon la revendication 1, ledit composant de délivrance de médicament (4) comprenant une couche de formulation de médicament (10) disposée entre un film dorsal et une membrane limitant le débit (130).

5. Système selon la revendication 1, ladite couche de formulation de médicament (10) disposée entre un film dorsal et une membrane ne limitant pas le débit (130).

6. Système selon la revendication 1, où ledit composant de régulation de la température (20) et ledit composant de délivrance de médicament (4) sont isolés l'un de l'autre de sorte que des vapeurs ne sont pas transférées entre les composants dans un emballage commun.

7. Système selon la revendication 1, ledit composant de délivrance de médicament (4) comprenant un médicament choisi dans le groupe de fentanyl, sufentanil, carfentanil, testostérone, lidocaïne, tétracaïne, prilocaïne, lopivacaïne, bupivacaïne, procaïne, flurbiprofène, naproxène, ibuprofène, scopolamine, nicotine et dexamethasone.

8. Système selon la revendication 1, ledit composant de délivrance de médicament (4) comprenant un médicament choisi dans les classes des analgésiques, agents anti-inflammatoires, stéroïdes, androgènes, estrogènes, hormones, agents antiviraux, agents anti-asthme, agents cardiovasculaires, agents antihypertension, antidépresseurs et inhibiteurs de cox-2.

9. Système selon la revendication 3, où le nombre choisi de trous (32) sont couverts de manière amovible avec des couvercles de trou limitant le débit (54).

10. Système selon la revendication 1, où le film formant barrière soudé au bord est séparable manuellement.

11. Procédé pour préparer un système de délivrance de médicament dermique employant de la chaleur régulée selon la revendication 1, le procédé comprenant les étapes de:
disposition d'une couche de formulation de médicament (10) dans un réservoir de formulation (40) d'un plateau (8) ayant un bord plat (44), le plateau (8) étant sensiblement imperméable à l'alcool et l'eau;
fixation de la couche de formulation de médicament (10) dans le plateau (8) à un applicateur de médicament;
fixation d'un composant de régulation de la température (20) à l'applicateur de médicament ; et
isolement de la couche de formulation de médicament (10) dans le plateau (8) avec un film formant barrière (16) qui couvre la couche de formulation de médicament (10) et est soudé au bord (44) du plateau (8) de sorte que le composant de régulation de la température (20) et la couche de formulation de médicament (10) sont isolés l'un de l'autre, le composant de régulation de la température (20) fixé à un côté supérieur du film formant barrière (16) et la couche de formulation de médicament (10) fixée à un côté inférieur du film formant barrière.

12. Procédé selon la revendication 11, où l'étape de fixation de la couche de formulation de médicament (10) comprend:
l'imprégnation d'une couche de matière de type étoffe avec un sel de sodium d'acide borique; et
la mise en contact de ladite matière de type étoffe avec ladite formulation, ladite formulation contenant du poly(alcool vinylique), pour amener la formulation de médicament à se solidifier sous forme d'un gel sur la matière de type étoffe.

13. Procédé selon la revendication 12, où l'étape d'isolement de la couche de formulation de médicament comprend:
la mise en place d'un film formant barrière thermosoudable (16) au-dessus de la matière de type étoffe et le soudage dudit film formant barrière (16) au plateau (8) et à la matière de type étoffe.

14. Procédé selon la revendication 13, où une partie du film formant barrière (16) n'est pas soudée sur le plateau (8) pour former un bord directeur pour la séparation.

15. Procédé selon la revendication 13, ladite couche de formulation de médicament (10) comprenant un médicament choisi dans le groupe de fentanyl, sufentanil, carfentanil, testostérone, lidocaïne, tétracaïne, prilocaïne, lopivacaïne, bupivacaïne, procaïne, flurbiprofène, naproxène, ibuprofène, scopolamine, nicotine et dexamethasone.

16. Procédé selon la revendication 13, ladite couche de formulation de médicament (10) comprenant un médicament choisi dans les classes des analgésiques, agents anti-inflammatoires, stéroïdes, androgènes, estrogènes, hormones, agents antiviraux, agents anti-asthme, agents cardiovasculaires, agents antihypertension, antidépresseurs et inhibiteurs de cox-2.
